Europäisches Patentamt

European Patent Office

Office européen des brevets

(19)

(11) Veröffentlichungsnummer: **0 451 823 A2**

## (12) EUROPÄISCHE PATENTANMELDUNG

(21) Anmeldenummer: 91105702.4

(22) Anmeldetag: 10.04.91

(51) Int. Cl.5: **C12N 15/85**, C12N 15/12, C07H 21/04, C12P 21/00

(30) Priorität: 11.04.90 DE 4011751
19.04.90 DE 4012526

(43) Veröffentlichungstag der Anmeldung:
16.10.91 Patentblatt 91/42

(84) Benannte Vertragsstaaten:
AT BE CH DE ES FR GB IT LI NL SE

(71) Anmelder: **Consortium für elektrochemische Industrie GmbH**
**Zielstattstrasse 20**
**W-8000 München 70(DE)**

(72) Erfinder: **Hartl, Peter, Dr. Dipl.-Chem.**
**Kruenerstrasse 89**
**W-8000 München 70(DE)**
Erfinder: **Brem, Gottfried, Prof. Dr. Dr.**
**Larezhausen**
**W-8893 Hilgertshausen(DE)**

(54) **DNA-Konstrukte zur Expression von Proteinen in der Milchdrüse transgener Säugetiere.**

(57) Die Erfindung betrifft ein rekombinantes DNA-Konstrukt zur Expression von Proteinen in der Milchdrüse transgener Säugetiere und nachfolgender Sekretion in die Milch, das die folgenden DNA-Bereiche in der angegebenen Reihenfolge und in funktioneller Verknüpfung zueinander enthält:

(1) eine Transkriptions-Kontrollregion aus einem oder mehreren spezifisch in der Milchdrüse aktivierten Genen,

(2) ein erster DNA-Sequenzbereich, der nicht translatiert wird,

(3) eine DNA-Sequenz, die für ein Signalpeptid kodiert, das eine Sekretion in die Milch erlaubt,

(4) eine DNA-Sequenz, welche die genetische Information für ein heterologes Peptid oder ein Protein enthält und

(5) eine DNA-Sequenz, die eine Polyadenylierungsstelle und ein Transkriptions-Terminationssignal enthält.

Die Erfindung betrifft weiterhin einen rekombinanten Vektor, der ein solches Konstrukt enthält, sowie Verfahren zur Gewinnung von Proteinen aus der Milch transgener Tiere, in deren Genom ein erfindungsgemäßes DNA-Konstrukt integriert ist.

Rank Xerox (UK) Business Services

Seit langem ist eine Gewinnung von Proteinen wie z.B. Albumin, Rennin, Interleukinen, Interferonen, Blutgerinnungsfaktoren, Insulin, Wachstumshormonen oder Somatostatinen aus Körperflüssigkeiten oder Gewebeextrakten von Mensch und Tier bekannt. Ebenso bekannt sind die Probleme, die dabei auftreten: virale Infektionen (AIDS, Hepatitis) oder Immunreaktionen (Insulin). Weiterhin ist in vielen Fällen auch eine Befriedigung der Nachfrage mit diesen Verfahren der Proteingewinnung nicht möglich (Insulin aus Schweinen, Rennin aus Kälbern).

Eine weitere Möglichkeit zur Gewinnung von Proteinen besteht in der chemischen Vollsynthese. Dies ist jedoch nur bei Proteinen mit weniger als 50 Aminosäuren möglich. Ein weiterer Nachteil dieses Verfahrens beruht auf der falschen Faltung und dem Fehlen der Glykosylierung bei chemisch synthetisierten Proteinen.

Anreicherungsverfahren von rekombinanten Proteinen aus Bakterien und Hefezellkulturen haben den Nachteil einer geringen Ausbeute, oftmals sind auch die Proteine falsch gefaltet (inclusion bodies), besitzen keine (Bakterien) oder die falsche (Hefen) Glykosylierung oder sie können aufgrund der Konstruktionsmöglichkeiten nur mit einer veränderten Aminosäurenzusammensetzung hergestellt werden. Desweiteren enthalten die zur Herstellung verwendeten Mikroorganismen oft toxische Substanzen, die eine Anwendung in der Lebensmittelindustrie nicht zulassen (E. coli), oder bei der medizinischen Anwendung zu Immunreaktionen führen können. Diese Probleme ziehen kostenintensive Verfahren zur Aufreinigung und/oder Aktivierung der rekombinanten Proteine nach sich.

Eine weitere Möglichkeit beruht auf der Gewinnung von rekombinanten Proteinen aus Kulturen höherer Zellen (Abstammung: Mensch, Tier). Geringe Ausbeuten und mögliche Kontaminationen (DNA, Fremdproteine) führen auch bei dieser Art der Herstellung zu hohen Kosten.

In jüngster Zeit wurde die Produktion rekombinanter Proteine in der Milch transgener Tiere als Lösung dieser Probleme vorgeschlagen (R. Lathe et al., p. 91 bis p. 102 in: Exploiting New Technologies in Animal Breeding, Published by Oxford University Press 1986, eds. C. Smith, J.W.B. King and J.C. McKay). Die Erstellung transgener Tierlinien ist seit längerer Zeit möglich (Wagner et al., 1981, Proc. Natl. Acad. Sci. USA 78: 6376-80; Patente: WO 82/04443; WO 87/5325).

Die gewebespezifische Expression von Proteinen unabhängig von der Art der Proteine wurde nachgewiesen (Übersicht: Palmiter et Brinster, 1986, Ann. Rev. Genet. 20: 465-499). Auch die spezifische Expression von Proteinen in der Milchdrüse transgener Tiere ist bekannt (Stewart et al. 1984, Cell 38: 627-37; Gordon et al. 1987, BIO/TECHNOLOGY 5: 1183-87; Pittius et al. 1988, Proc. Natl. Acad. Sci. USA 85: 5874-78; Clark et al. 1989, BIO/TECHNOLOGY 7: 487-92; Andres et al. 1987, Proc. Natl. Acad. Sci. USA 84: 1299-1303; WO 88/10118; WO 88/00239; EP 0279 582, WO 88/01648; EP 0264 166).

So beansprucht die EP-A-0 264 166 eine DNA-Sequenz, die ein Gen enthält, welches für ein Protein kodiert, wobei das Gen auf der DNA-Sequenz unter transkriptioneller Kontrolle eines Milchprotein-Promotors aus Säugetieren ist, welcher nicht natürlicherweise die Transkription des Gens kontrolliert, wobei die DNA-Sequenz weiterhin einen Bereich enthält, der die Sekretion des Proteins ermöglicht.

Die WO 88/01648 beansprucht ein rekombinantes Expressionssystem, welches eine lactogen induzierbare regulatorische Region und eine strukturelle Region, die für ein heterologes Protein kodiert, enthält. Die regulatorische Region stammt vorzugsweise von α-Lactalbumin.

Die EP-A-0 279 582 beansprucht eine rekombinante DNA, die eine Promotorsequenz, eine Enhancer-Sequenz, eine Signalpeptidsequenz und eine kodierende Sequenz enthält, wobei die Promotor-, Enhancer- und Signalpeptid-Sequenz von mindestens einem Milchdrüsen-spezifischen Gen stammen und die Expression der kodierenden Sequenz in der Milchdrüse verbessern. Offenbart wird die Synthese des Ratten-$\beta$-Caseins und eines Chloramphenicolacetyltransferase-Fusionsproteins in der Milchdrüse der Maus.

Die WO 88/10118 beansprucht eine DNA-Sequenz, die einen milchspezifischen Promotor oder einen spezifisch im Brustgewebe aktivierten Promotor operativ mit einer DNA-Sequenz verknüpft enthält, die für ein rekombinantes Protein kodiert, wobei die Verknüpfung über eine DNA-Sequenz geschieht, die für ein Signalpeptid kodiert, das die Sekretion und Reifung des rekombinanten Proteins im Brustgewebe bewirkt. Gezeigt wird eine geringe Expression eines t-PA-Fusionsproteins in der Maus (0,2 bis 0,5 μg/ml in der Milch) und im Schaf.

Die zur Expression von Proteinen in der Milchdrüse benutzten DNA-Konstrukte führten bisher in allen Fällen zu geringen Ausbeuten oder/und zu Fusionsproteinen, so daß im wesentlichen die zuvor beschriebenen Nachteile der bekannten Verfahren zur Proteingewinnung nicht beseitigt werden. Daher war es die Aufgabe der vorliegenden Erfindung, nach einem verbesserten Expressionssystem zur Gewinnung von Proteinen auch ohne Fusionsanteil aus der Milch transgener Tiere zu suchen, das hohe Ausbeuten des gewünschten Proteins liefert sowie eine Aufreinigung des Proteins erleichtert.

Durch die vorliegende Erfindung werden diese Probleme gelöst. Die Erfindung beruht auf einem rekombinanten DNA-Konstrukt, das verschiedene Sequenzbereiche enthält, die einerseits zu einer hohen Expression gewünschter Proteine oder Peptide in der Milchdrüse von transgenen Tieren führen und andererseits die Sekretion der gebildeten Proteine in die Milch veranlassen. Die Aktivierung des rekombinanten DNA-Konstrukts ist ausschließlich auf die Milchdrüse beschränkt.

Die verwendeten DNA-Sequenzbereiche werden erfindungsgemäß zu einem gewebespezifisch in der Milchdrüse aktiven DNA-Konstrukt zusammengefügt, das aus Promotor-, Enhancer- und Signalpeptidsequenzen sowie aus spezifischen 5'- und 3'-nichttranslatierten und -transkribierten DNA-Bereichen besteht. Teile der verwendeten transkribierten DNA-Bereiche werden bei der Reifung des Primärtranskripts zur mRNA durch Spleißvorgänge entfernt. Desweiteren enthält das DNA-Konstrukt auch einen für ein heterologes Protein oder ein Peptid kodierenden Bereich, der gegebenenfalls durch Introns unterbrochen sein kann. Diese DNA-Sequenzen werden funktionell zu einer operativen Einheit verknüpft, die in der Milchdrüse positiv reguliert ist und zu einer hohen Proteinexpression führt. So bewirkt die Integration eines erfindungsgemäßen DNA-Konstrukts in die Keimbahn von Kaninchen oder Rindern, daß die Milch der resultierenden transgenen Tiere eine hohe Konzentration der rekombinanten Proteine aufweist.

Ein Gegenstand der Erfindung ist somit ein rekombinantes DNA-Konstrukt zur Expression von Proteinen in der Milchdrüse transgener Säugetiere und nachfolgender Sekretion in der Milch, das die im folgenden aufgeführten DNA-Bereiche in der angegebenen Reihenfolge und in funktioneller Verknüpfung zueinander enthält:

(1) eine Transkriptions-Kontrollregion aus einem oder mehreren spezifisch in der Milchdrüse aktivierten Genen,

(2) ein erster DNA-Sequenzbereich, der nicht translatiert wird,

(3) eine DNA-Sequenz, die für ein Signalpeptid kodiert, das eine Sekretion in die Milch erlaubt,

(4) eine DNA-Sequenz, welche die genetische Information für ein heterologes Peptid oder ein Protein enthält und

(5) eine DNA-Sequenz, die eine Polyadenylierungsstelle und ein Transkriptions-Terminationssignal enthält.

Der Begriff "funktionelle Verknüpfung" bedeutet, daß die einzelnen DNA-Sequenzen jeweils auf solche Weise miteinander verknüpft sind, so daß die Expression des erfindungsgemäßen DNA-Konstrukts ein Vorläufer-Peptid oder -Protein ergibt, aus dem nach Abspaltung einer Signalsequenz direkt das gewünschte Peptid oder Protein, sofern gewünscht, ohne jeden Fusionsanteil entsteht. Die Verknüpfung der einzelnen DNA-Abschnitte zum erfindungsgemäßen DNA-Konstrukt erfolgt im Einzelfall mittels molekularbiologischer Techniken, die dem Fachmann auf diesem Gebiet an sich bekannt sind. In einigen Fällen ist es möglich, Fragmente von Genen direkt durch Ligation miteinander zu verknüpfen. In den meisten Fällen ist es jedoch zur Herstellung eines erfindungsgemäßen DNA-Konstrukts erforderlich, Fragmente von Genen über synthetisch hergestellte DNA-Fragmente zu verknüpfen. Weiterhin ist es auch möglich, das gesamte DNA-Konstrukt oder wesentliche Teile davon durch die Verknüpfung synthetischer DNA-Fragmente zu konstruieren.

Der Begriff "Transkriptions-Kontrollregion" bedeutet einen Promotor- und Enhancer-Bereich, der die gewebespezifische Initiation und Aktivierung der Transkription in der Milchdrüse eines transgenen Tieres ermöglicht. Die Transkriptions-Kontrollregion stammt dabei von mindestens einem speziell in der Milchdrüse aktivierten Gen, wie z.B. vom $\alpha$- oder $\beta$-Lactoglobulingen oder von einem Mitglied der Caseingen-Familie, z.B. vom $\alpha$-$S_1$-Caseingen. Besonders bevorzugt ist die Verwendung der Transkriptions-Kontrollregion des $\alpha$-$S_1$-Caseingens, insbesondere vom Rind oder vom Kaninchen. Die Transkriptions-Kontrollregion muß nicht aus einem einzigen Gen stammen, es ist auch möglich, die zur gewebespezifischen Expression des DNA-Konstrukts erforderlichen Regulationselemente aus zwei oder mehreren Genen zu entnehmen.

Auf diese Transkriptions-Kontrollregion folgt ein erster DNA-Sequenzbereich, der nicht translatiert wird. Vorzugsweise enthält dieser Bereich eine Sequenz, die für das nichttranslatierte Exon I eines Gens aus der Caseinfamilie ($\alpha$-$S_1$, $\alpha$-$S_2$, $\beta$, K-Casein) kodiert, wobei besonders bevorzugt das Exon I aus dem $\alpha$-$S_1$-Caseingen verwendet wird. Es folgt dann vorzugsweise eine DNA-Sequenz, die für ein erstes Intron kodiert, wobei die DNA-Sequenz am Exon/Intron-Übergang eine native Splice-Stelle eines Milchdrüsen-spezifischen Gens, besonders bevorzugt des $\alpha$-$S_1$-Caseingens sein soll. Der restliche Bereich des Introns muß nicht aus einem Milchdrüsenspezifischen Gen stammen, vorzugsweise verwendet man jedoch das erste Intron aus dem $\alpha$-$S_1$-Caseingen.

Die Verknüpfung der für das zu exprimierende Protein kodierenden DNA-Sequenz mit den spezifisch in der Milchdrüse zu hoher Expression führenden DNA-Sequenzen geschieht über eine DNA-Sequenz, die für ein Signalpeptid kodiert, welche zur Sekretion des Proteins aus den Zellen der Milchdrüse in die Milch führt. Erfindungsgemäß werden vorzugsweise entweder die eigenen, für ein Signalpeptid kodierenden DNA-Sequenzen des zu exprimierenden Proteins oder die für ein Signalpeptid kodierenden DNA-Sequenzen eines milchdrüsenspezifischen Proteins verwendet. Besonders bevorzugt werden hier die Signalsequenzen des $\alpha$-$S_1$-Caseingens von Rind oder Kaninchen eingesetzt. Die Sequenz des Signalpeptides ist so mit der für das Protein kodierenden Sequenz verknüpft, daß beim Export des Proteins aus den Milchdrüsenzellen dieses Signalpeptid exakt an der Verknüpfungsstelle abgespalten wird, so daß in der Milch das gewünschte Protein in der entsprechenden aktiven oder inaktiven Form vorliegt.

Das erfindungsgemäße DNA-Konstrukt enthält ferner die genetische Information für ein zu exprimierendes heterologes Protein oder Peptid. Der Begriff "heterolog" bedeutet dabei, daß sich die kodierende DNA-Sequenz unter Kontrolle einer fremden Transkriptions-Kontrollregion befindet. Die Expression der kodierenden DNA-Sequenzen führt zur Synthese eines inaktiven Propeptides, eines reifen, aktiven Proteins und, sofern gewünscht, auch eines Fusionsproteins in der Milchdrüse. Die Wahl einer dieser Möglichkeiten kann jeweils nach den Eigenschaften und dem Verwendungszweck des exprimierten Proteins erfolgen. Milchverändernde Proteine werden bevorzugt in einer inaktiven Form exprimiert. So wird Rennin (oder Chymosin, EC 3.4.23.4) bevorzugt in der inaktiven Form Prorennin (oder Prochymosin) gebildet. Proteine, welche keine Veränderung der Milch bewirken, z.B. menschlicher IGF-I (Insulin-ähnlicher Wachstumsfaktor I) und menschliches Albumin dagegen werden bevorzugt in der reifen Form gebildet. Die Synthese eines Fusionsproteins kann z.B. von Nutzen sein, wenn der Fusionsanteil eine leichtere Reinigung des Produkts (z.B. durch Affinitätschromatographie) ermöglicht und anschließend durch eine spezifische Spaltung (z.B. chemisch oder proteolytisch) entfernt werden kann.

Als genetische Information für das entsprechende Protein kann man entweder genomische DNA-Sequenzen, die entsprechenden cDNA-Sequenzen oder eine Kombination dieser Sequenzen verwenden. Ebenso sind aber auch über Oligonukleotidsynthese hergestellte, totalsynthetische, für ein Protein kodierende Sequenzen geeignet. Die Erfindung beinhaltet weiterhin die Verwendung beliebiger Kombinationen aus genomischen DNA-Sequenzen, cDNA-Sequenzen und chemisch synthetisierten DNA-Sequenzen, die für ein Protein oder verschiedene Proteine kodieren und zu dem gewünschten Produkt führen. Vorzugsweise achtet man bei der Konstruktion synthetischer DNA-Sequenzen auf die dem Fachmann bekannte Benutzungshäufigkeit der Nukleinsäure-Codons in der zur Erzeugung transgener Tiere jeweils verwendeten Tierart.

An das Stopcodon der für das zu exprimierende Protein kodierenden DNA-Sequenz schließt sich entweder unmittelbar eine DNA-Sequenz an, welche eine Polyadenylierungsstelle und Transkriptions-Terminations-Stelle enthält, oder bevorzugt ein zweiter nicht-translatierter DNA-Sequenzbereich, der einen Exon/Intron-Übergang enthält. Vorzugsweise handelt es sich dabei um eine DNA-Sequenz, die den Exon/Intron-Übergang zwischen dem vorletzten Exon und dem letzten Intron eines Gens der Caseingen-Familie, besonders bevorzugt dem $\alpha$-$S_1$-Caseingen enthält.

Diesem Exon/Intron-Übergang folgt vorzugsweise eine DNA-Sequenz, die für ein weiteres Intron kodiert. Es folgt dann darauf die DNA-Sequenz, die eine Polyadenylierungsstelle und ein Transkriptions-Terminations-Stelle enthält.

Polyadenylierungsstelle und Transkriptions-Terminations-Stelle der in der Erfindung verwendeten DNA-Konstrukte können dem eigenen Genbereich des zu synthetisierenden Proteins oder einem milchdrüsenspezifischen Gen entnommen werden. Vorzugsweise wird die Polyadenylierungsstelle des $\alpha$-$S_1$-Caseingens verwendet. Erfindungsgemäß können auch künstliche, chemisch synthetisierte Terminationssequenzen (z.B. beschrieben in Levitt et al 1989, Genes and Development 3: 1019-1025) verwendet werden.

Im Anschluß an die Polyadenylierungsstelle werden vorzugsweise in den verwendeten DNA-Konstrukten weitere nichttranskribierte und nichttranslatierte 3'-DNA-Sequenzen benutzt, welche eine Expression fördern. Diese Sequenzen stammen aus dem ursprünglichen, von dem zu synthetisierenden Protein stammenden Genbereich oder von einem milchdrüsenspezifischen Gen. Vorzugsweise wird hierzu der $\alpha$-$S_1$-Caseingenbereich des Rindes oder des Kaninchens verwendet.

Erfindungsgemäß bevorzugt werden die DNA-Bereiche zu einer Expressionskassette zusammengesetzt, deren 5'- und 3'-Ende durch Anfügen von Oligonukleotiden so modifiziert ist, daß die Abtrennung dieser Expressionskassette von einem größeren DNA-Komplex durch vollständige Spaltung mit Restriktionsenzymen möglich ist, ohne daß dabei die Expressionskassette zerstört wird. Dabei verwendet man vorzugsweise Schnittstellen für Restriktionsenzyme, welche eine eukaryotische DNA statistisch selten schneiden. Dadurch ist es möglich, die Expressionskassette von DNA-Bereichen abzuspalten, die zur Replikation in Bakterien befähigt sind und eine genomische Integration bei der Erzeugung transgener Tiere stören. Vorzugsweise werden solche Enzyme und ihre Schnittstellen verwendet, die 8 Basenpaare als Erkennungssequenz besitzen oder/und in ihrer Erkennungsstelle mindestens einmal, besser mehrmals, die in eukaryontischer DNA seltene Basenfolge CpG besitzen wie z.B. SalI, NotI, SacII, PvuI. So spalten Enzyme mit einer Erkennungssequenz, die zweimal die Basenfolge CpG enthält, eine eukaryotische DNA statistisch nur ca. alle 150 kb. Mit Hilfe der erfindungsgemäßen Expressionskassette kann jedes beliebige Protein in aktiver oder inaktiver Form, in reifer Form oder als Bestandteil eines Vorläufer- bzw. eines Fusionsproteins in hoher Ausbeute spezifisch in der Milch transgener Tiere gebildet werden.

Weiterhin ein Gegenstand der vorliegenden Erfindung ist ein rekombinanter Vektor, der ein erfindungsgemäßes DNA-Konstrukt enthält. Dabei ist die Art dieses Vektors beliebig, d.h. es kann ein eukaryontischer oder ein prokaryontischer Vektor sein, er kann zur Integration in ein Genom oder zur extrachromosomalen Replikation fähig sein. Alle derartige Vektoren sind dem Fachmann auf dem Gebiet der Molekularbiologie bekannt. Man kann den rekombinanten Vektor zur Vermehrung der erfindungsgemäßen DNA-Konstrukte verwenden, man kann jedoch auch einen rekombinanten Vektor herstellen, der zur Integration von erfindungsgemäßen DNA-Konstrukten in die Keimbahn von Säugetieren geeignet ist, z.B. ein replikationsdefekter Retrovirus. (Übersicht: Palmiter et Brinster, (1986), Ann. Rev. Genet. 20:465-499).

Weiterhin ein Gegenstand der vorliegenden Erfindung ist ein Verfahren zur Gewinnung von Proteinen aus der Milch transgener Säugetiere, wobei man ein erfindungsgemäßes rekombinantes DNA-Konstrukt oder einen rekombinanten Vektor, welcher das erfindungsgemäße DNA-Konstrukt enthält, in die Keimbahn eines Tieres einbringt, wobei ein transgenes Tier entsteht, und das Protein auf übliche Weise aus der Milch des Tieres oder seiner weiblichen Nachkommen isoliert.

Für das erfindungsgemäße Verfahren ist es nicht entscheidend, auf welche Art die transgenen Tiere erzeugt werden. Bevorzugt werden die transgenen Tiere durch Mikroinjektion in den Pronukleus einer Keimzelle erzeugt. Es ist jedoch auch möglich, andere dem Fachmann bekannte Verfahren zur Herstellung transgener Tiere zu verwenden. So kann z.B. ein retroviraler Vektor konstruiert werden, der das erfindungsgemäße Konstrukt enthält, und den man dann in den Genom eines Tieres einbringen kann. Die Embryonen oder die geborenen Tiere werden auf Integration der Konstrukte überprüft. Positive, mindestens eine vollständige Kopie des Expressionssystems enthaltende Tiere (Primärtiere) werden zur Aufzucht verwendet. Positive Tiere werden vermehrt und deren Nachkommen (F 1-Generation) ebenfalls auf Gehalt des Transgens überprüft. Üblicherweise wird das Transgen über die Keimbahn von den Primärtieren auf die F 1-Gernation nach den Mendelschen Vererbungsregeln übertragen.

Von positiven weiblichen Primärtieren wird in der Laktationsperiode die Milch auf Gehalt des Proteins überprüft. Von positiven männlichen Tieren werden weibliche F 1-Nachkommen oder weibliche Nachkommen einer späteren Generation bezüglich der Bildung des Proteins in ihrer Milch getestet. Positive Tiere, deren Nachkommen oder die selbst in ihrer Milch die rekombinanten Proteine bilden, werden durch Vermehrung zur Aufzucht stabiler Tierlinien verwendet.

Die rekombinanten Proteine werden aus der Milch nach üblicherweise bekannten, für das jeweilige Protein geeigneten Verfahren isoliert. Erfindungsgemäß wird die Isolation der rekombinanten Proteine erleichtert durch eine geeignete Auswahl der zur Erzeugung transgener Tiere verwendeten Ei- und Samenzellen. Dieses Auswahlverfahren wird dann durchgeführt, wenn normalerweise ein endogenes, zum rekombinanten Protein homologes, Protein in der Milch vorliegt. In diesem Fall werden Keimbahnzellen zur Erzeugung transgener Tiere herangezogen, die aufgrund genetischer Bedingungen das entsprechende endogene Protein nicht in der Milch bilden. Die genetischen Bedingungen sind entweder natürlicher Art oder können durch gezielte Manipulation erreicht werden. Vorzugsweise wird daher z.B. zur Gewinnung von menschlichem Serumalbumin aus der Milch transgener Tiere eine Variante gewählt, die kein eigenes Serumalbumin bildet (Esumi et al. (1982), Proc. Natl. Acad. Sci. USA 79:734-738; Inoue (1985), Hepatology 5:892-898).

Die Erfindung beinhaltet auch eine Auswahl von Keimbahnzellen zur Erzeugung transgener Tiere von einer Spezies, die in ihrer Milch bezüglich der Aminosäuresequenz ein zum rekombinanten Protein vollständig homologes Protein bildet. Diese Homologie kann natürlicher Art sein oder auf einer genetischen Variante beruhen. Vorzugsweise wird der reife, menschliche IGF-I in der Milch transgener Schweine und Rinder gebildet, deren endogener reifer IGF-I die identische Aminosäuresequenz besitzt.

Erfindungsgemäß ist auch die Gewinnung eines aus zwei oder mehreren Untereinheiten zusammengesetzten Proteins aus der Milch eines transgenen Tieres möglich. Dies geschieht so, daß man ein transgenes Tier verwendet, das zwei oder mehrere verschiedene erfindungsgemäße DNA-Konstrukte in seinem Genom integriert besitzt. Somit beinhaltet die Erfindung auch ein Verfahren zur Herstellung transgener Tiere, die zwei oder mehrere verschiedene Fremdgene in ihrem Genom integriert besitzen. Dafür existieren zwei Möglichkeiten. So kann man zwei transgene Tiere der gleichen Art, die aber jeweils ein verschiedenes Fremdgen, vorzugsweise in Form eines erfindungsgemäßen DNA-Konstrukts in ihrem Genom integriert besitzen, auf übliche Weise (z.B. durch Mikroinjektion in die Keimzellen) herstellen, diese Tiere oder deren Nachkommen miteinander kreuzen und aus den Nachkommen dieser Kreuzung diejenigen transgenen Tiere auswählen, welche gleichzeitig für die verschiedenen Fremdgene beider Elterntiere positiv sind. Andererseits kann man auch ein transgenes Tier mit zwei oder mehreren Fremdgenen in seinem Genom herstellen, indem man ein Fremdgen, vorzugsweise ein erfindungsgemäßes DNA-Konstrukt in die Keimbahn eines Tieres einbringt, das bereits eines oder mehrere andere Fremdgene in seinem Genom integriert besitzt. Auf diese Weise sind transgene Tiere herstellbar, die beliebig viele aktive Fremdgene in ihrem Genom integriert besitzen. Bei Verwendung von erfindungsgemäßen DNA-Konstrukten erfolgt eine gleichzeitige Sekretion von mehreren Proteinen oder Peptiden in die Milch, so daß sich gegebenenfalls dort ein aus zwei oder mehreren verschiedenen Untereinheiten zusammengesetzter Proteinkomplex assemblieren kann.

Weiterhin ist somit ein Gegenstand der vorliegenden Erfindung die Milch eines transgenen Tieres, das ein oder mehrere rekombinante erfindungsgemäße DNA-Konstrukte in seinem Genom integriert enthält.

Die Erfindung beinhaltet die Expression jeglicher Proteine. Dies können inaktive Vorläuferproteine, aktive reife Proteine oder Teilproteine eines größeren Proteinkomplexes sein sowie Teile eines Fusionsproteins. Allein die kodierende DNA-Sequenz bestimmt die Form des kodierenden Proteins. Bei erfindungsgerechter Verwendung der beschriebenen DNA-Sequenzen können z.B. Human- oder Tier-Serumalbumin, Urokinase, t-PA, Wachstumshormone, Interferone, Peptidhormone, Prorennin und andere Proteine gebildet werden.

Für die Verwendung erfindungsgemäß hergestellter Proteine besteht keine Einschränkung. Vorzugsweise werden sie im medizinischen bzw. pharmazeutischen Bereich oder in der Lebensmitteltechnik eingesetzt. Besonders geeignet ist die Verwendung für Zwecke in der Lebensmitteltechnologie, da die Begleitproteine des rekombinanten Proteins aus der Milch bei diesen Anwendungen nicht toxisch sind. Daher ist bei bestimmten rekombinanten Proteinen keine oder nur eine partielle Trennung von den Milchproteinen nötig.

Schließlich beinhaltet die Erfindung auch noch ein rekombinantes DNA-Konstrukt zur Herstellung einer Expressionskassette, das anstelle des DNA-Bereichs, welcher die genetische Information für ein heterologes Peptid oder ein Protein enthält, eine Klonierungsstelle zum Einbau eines derartigen DNA-Bereichs enthält. Bei dieser Klonierungsstelle kann es sich um die Schnittstelle eines Restriktionsenzyms handeln. Vorzugsweise handelt es sich um eine singuläre Restriktionsschnittstelle, d.h. um eine Schnittstelle, die nur einmal im gesamten DNA-Konstrukt vorkommt. Besonders bevorzugt enthält diese Klonierungsstelle die Schnittstellen für mehrere Restriktionsenzyme, wobei man günstigerweise solche Restriktionsenzyme auswählt, die im gesamten DNA-Konstrukt nur eine Schnittstelle besitzen.

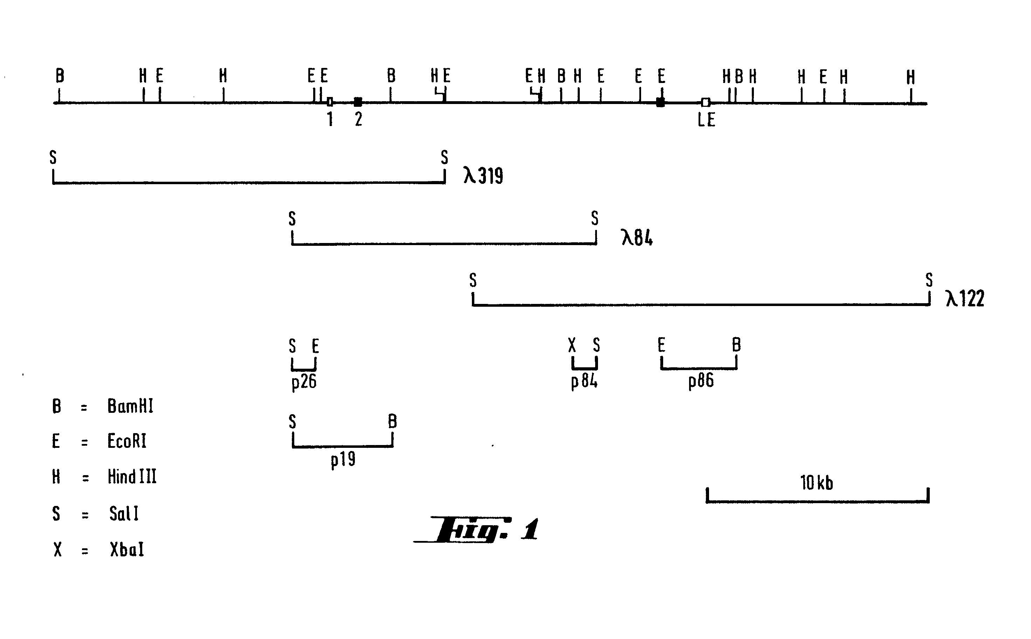

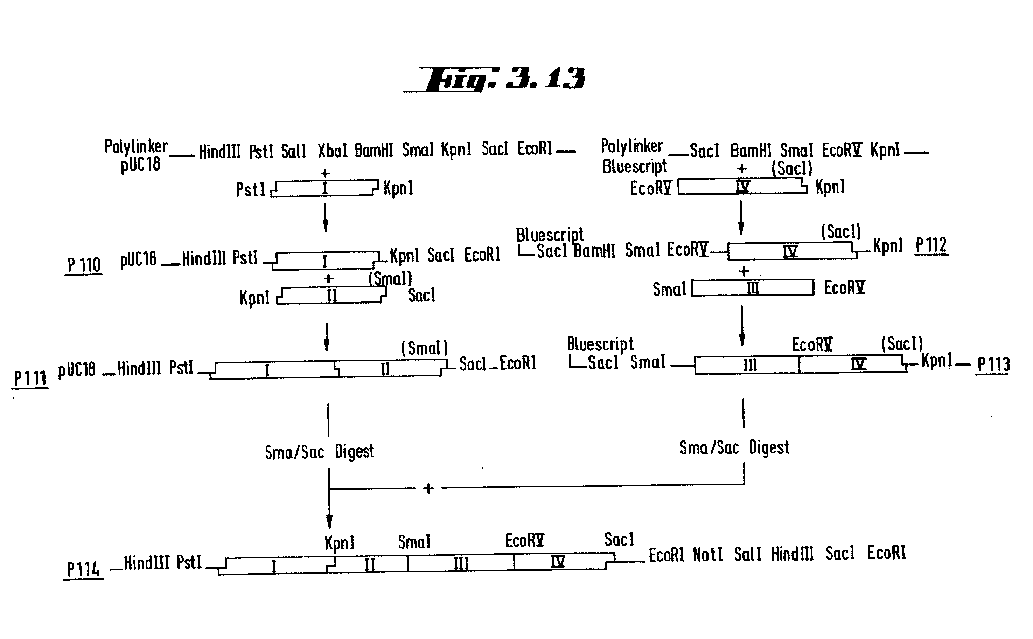

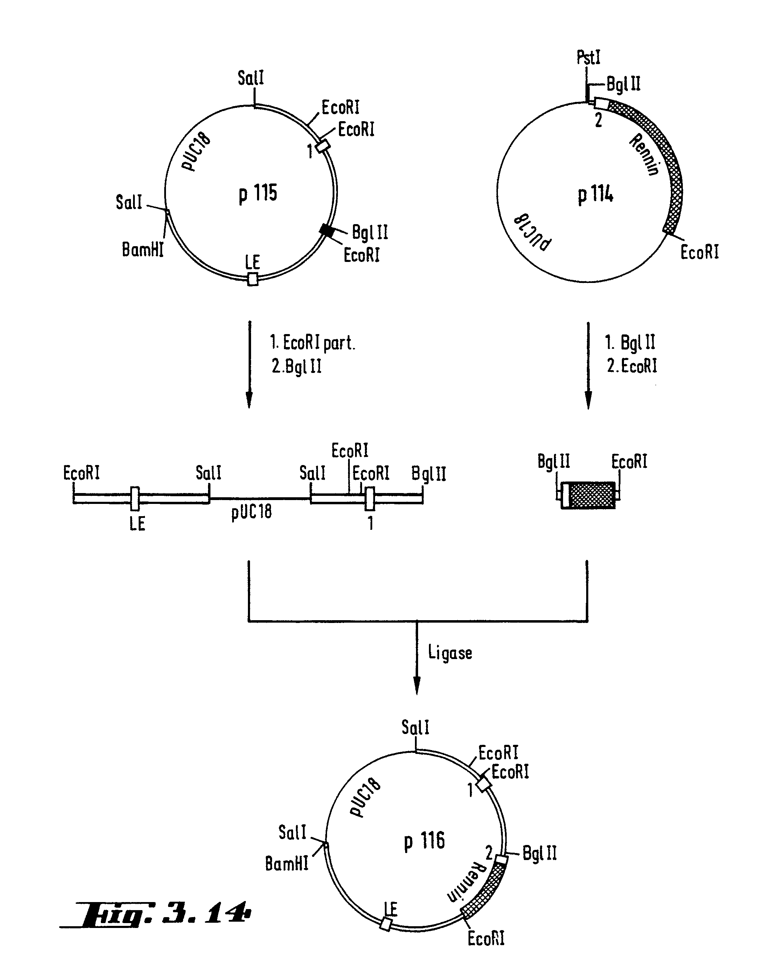

Die folgenden Beispiele sollen zusammen mit den Abbildungen 1 bis 5 die Erfindung besser verdeutlichen.

In den Abbildungen sind Exons sowie Konstrukte zueinander nicht maßstabgerecht wiedergegeben.

Es zeigen:

Abbildung 1:

α-S$_1$-Caseingenbereich des Rindes mit Subklonen, siehe Beispiel 1 (die Nukleotidsequenz des Konstrukts p19 ist in Anhang 1 dargestellt).

Abbildung 2:

Renningenbereich des Rindes mit Subklonen, s. Beispiel 2.

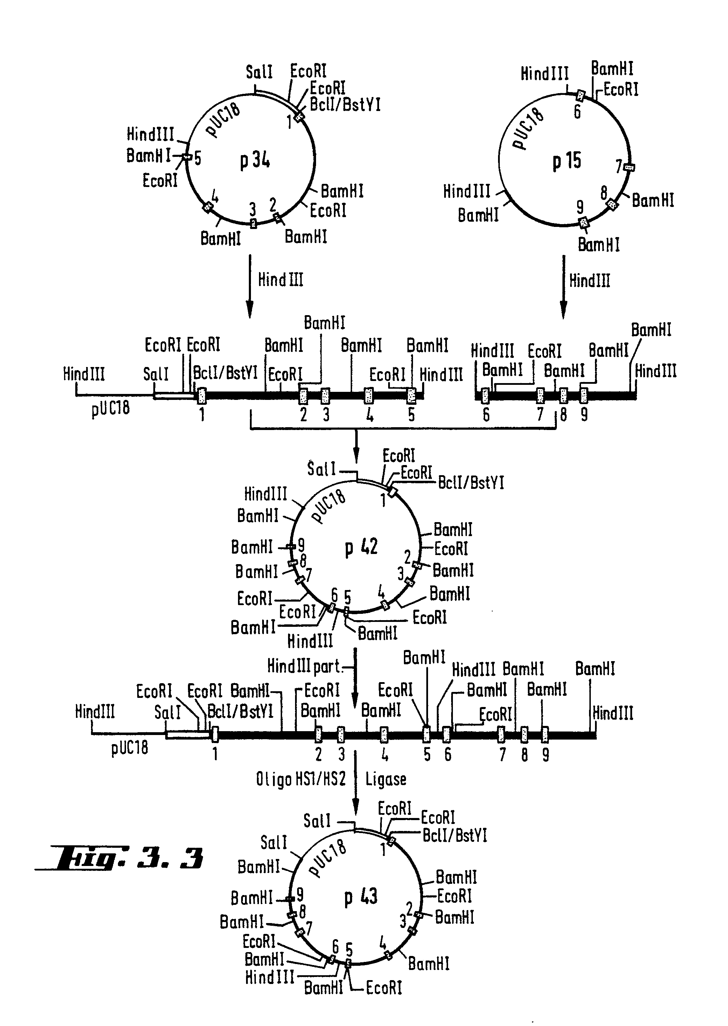

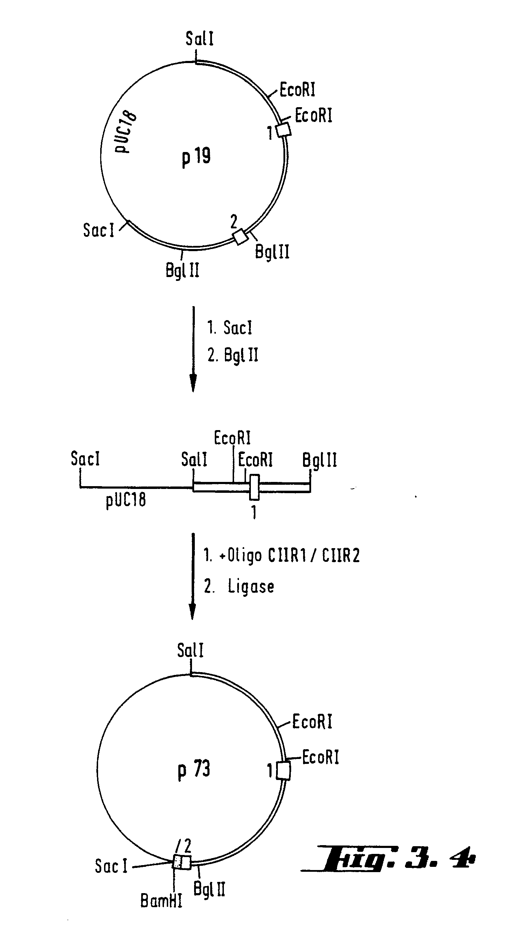

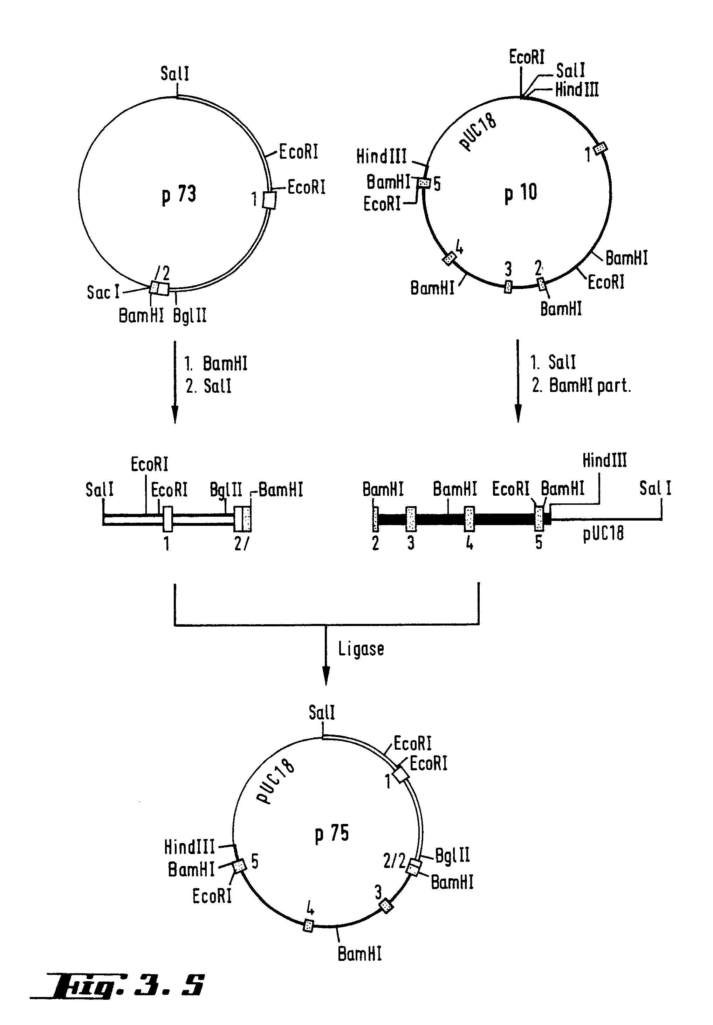

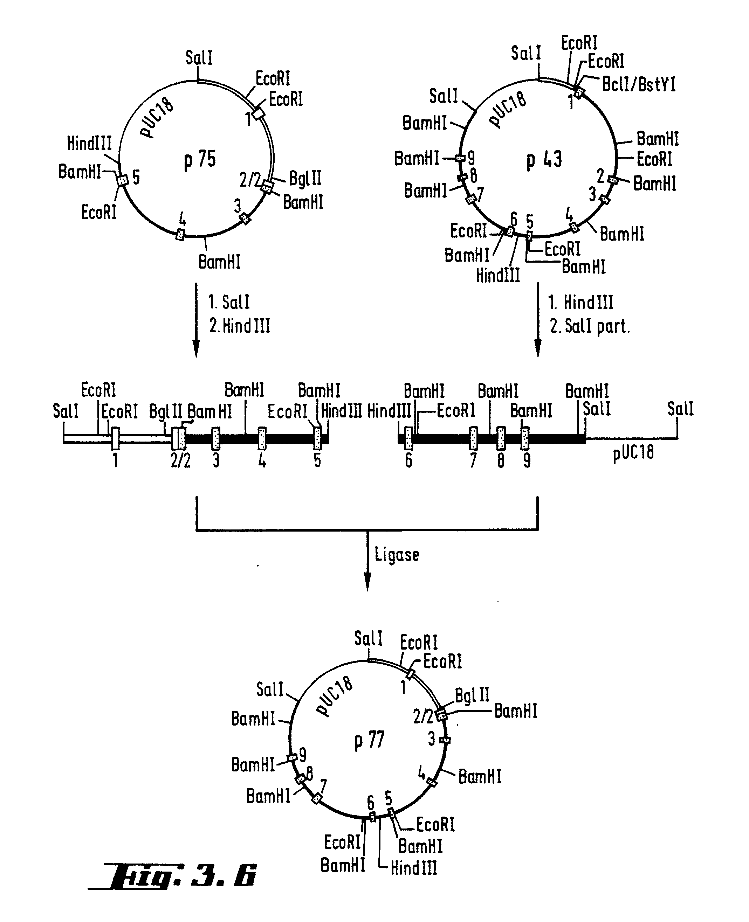

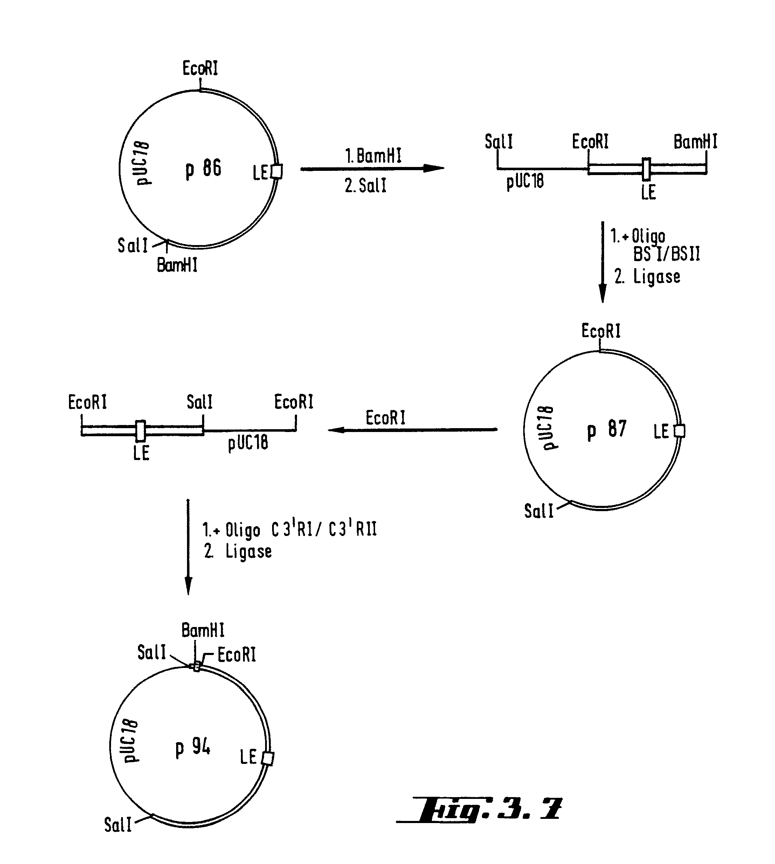

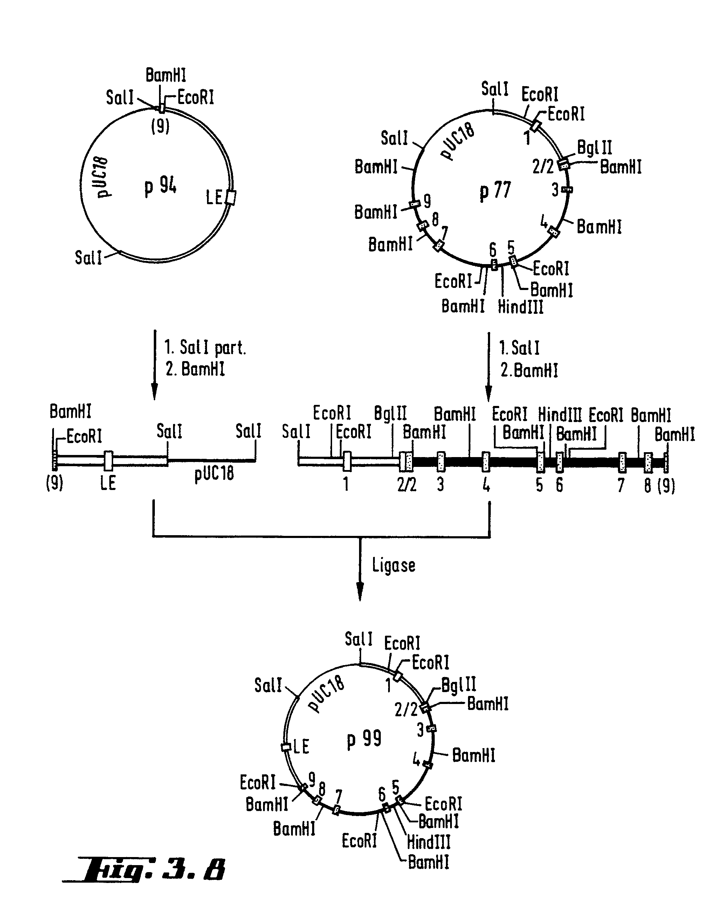

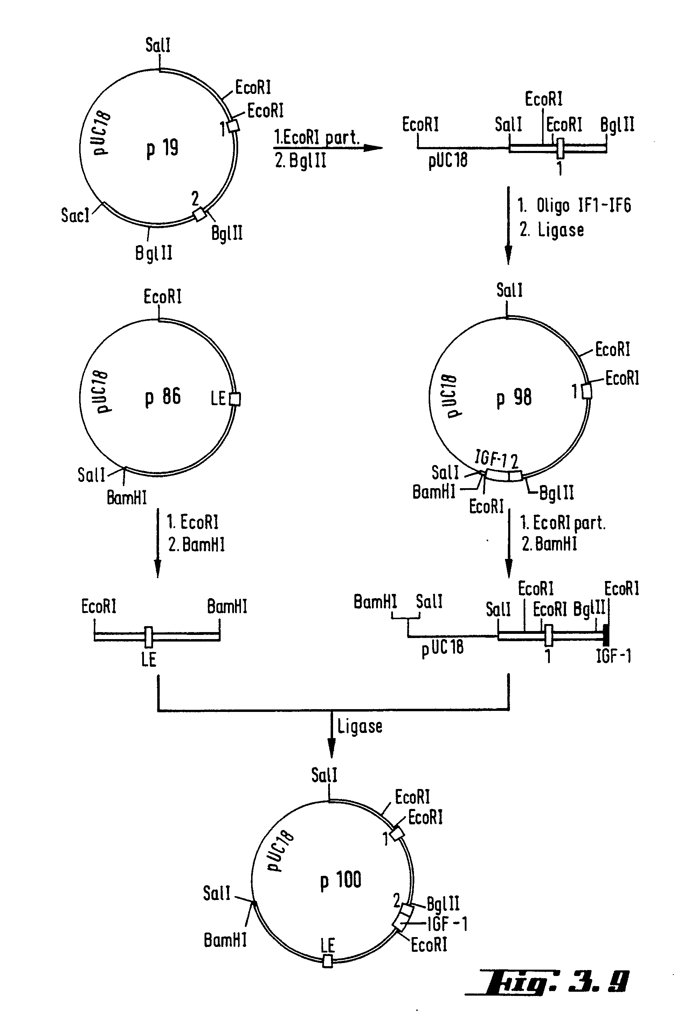

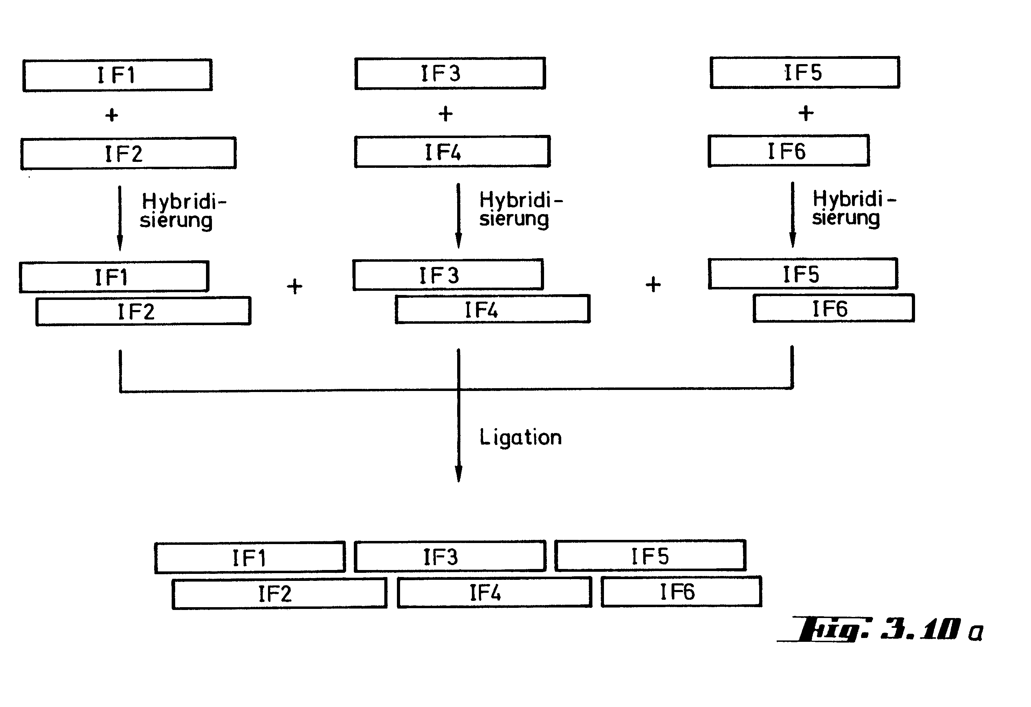

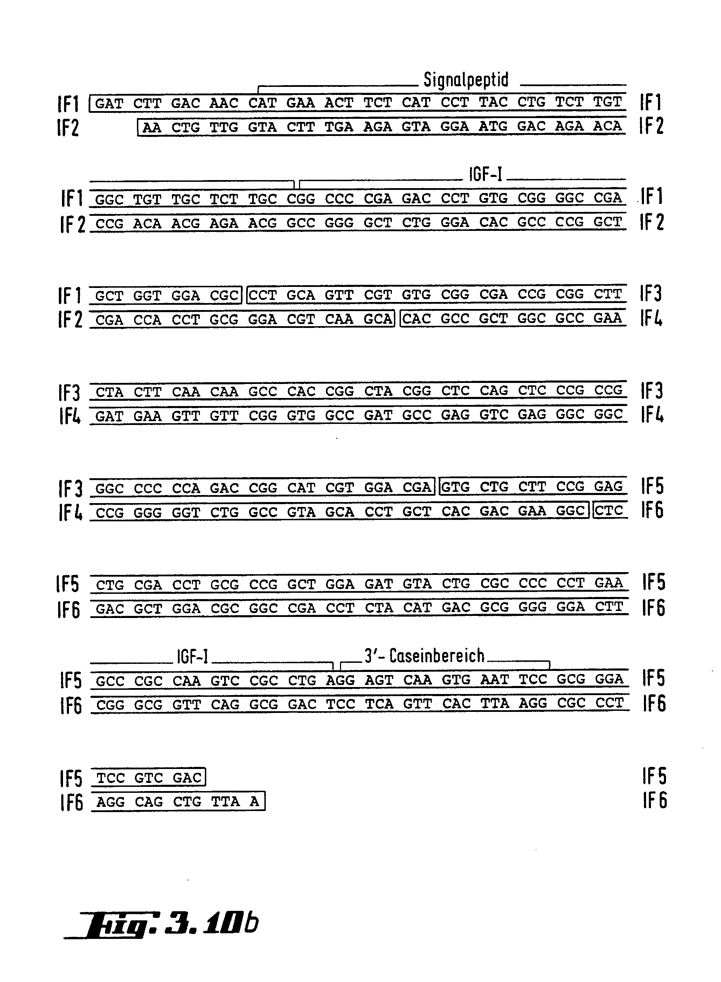

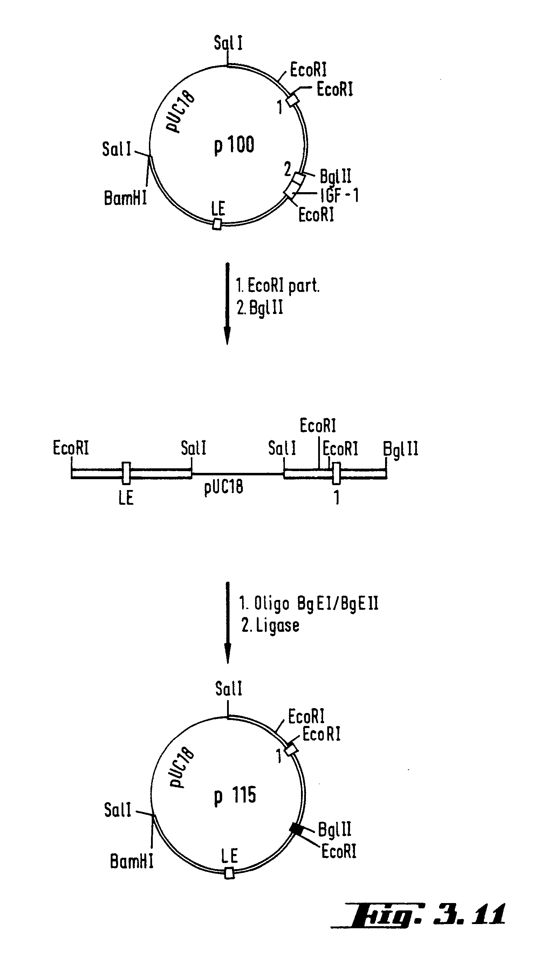

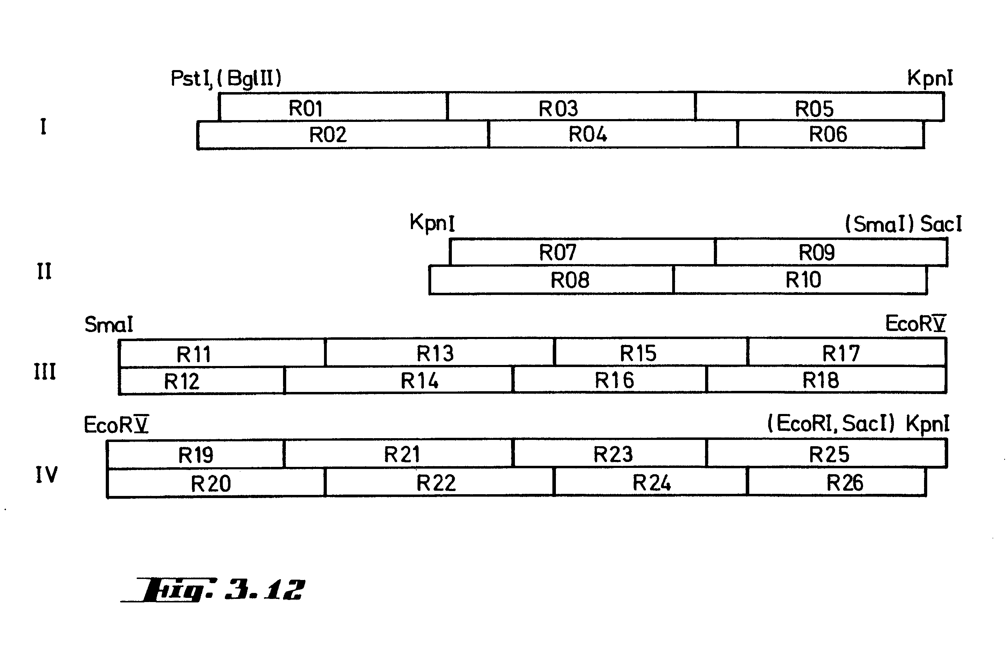

Für die Abbildungen 3.1 bis 3.14 bedeuten, sofern nichts anderes angegeben ist, die folgenden Symbole:

———— = Vektorbereich     LE = Letztes nichtkodierendes Exon des α-s1-Caseingens

▭ = α-s1-Caseingenbereich     ▯ = Exon des α-s1-Caseingens

▬▬ = Renningenbereich     ▤ = Exon des Renningens

Abbildungen 3.1 bis 3.3:

Verknüpfung des Bovinen α-S$_1$-Caseinpromotors mit dem Renningen, s. Beispiel 3a.

Abbildungen 3.4 bis 3.6:

Verknüpfung des Renningens mit dem α-S$_1$-Caseinpromotor über den für das Signalpeptid des α-S$_1$-Caseins kodierenden Bereich, s. Beispiel 3b.

Abbildungen 3.7 bis 3.8:

Verknüpfung des Renningens mit dem $\alpha$-$S_1$-Caseinpromotor,dem $\alpha$-$S_1$-Signalpeptid sowie 3'-nichttranslatierten Sequenzen des $\alpha$-$S_1$-Caseingens, s. Beispiel 3c.

Abbildungen 3.9 bis 3.10:

Expressionssystem für menschlichen IGF-I unter Kontrolle 5'- und 3'-regulierender DNA-Sequenzen aus dem bovinen $\alpha$-$S_1$-Caseingenbereich.

Abbildung 3.11:

Entwicklung einer Expressionskassette zur Synthese von Proteinen in der Milchdrüse transgener Tiere, siehe Beispiel 3e.

Abbildungen 3.12 bis 3.14:

Expressionssystem für Rennin unter Kontrolle regulierender 5,- und 3'-Sequenzen aus dem bovinen $\alpha$-$S_1$-Caseinbereich unter Verwendung eines synthetischen Renningens, siehe Beispiel 3f (die kodierende Nukleinsäuresequenz des synthetischen Renningens ist in Anhang 2a, die komplementäre Nukleinsäuresequenz in Anhang 2b dargestellt).

Beispiele

Methoden wurden - falls nicht besonders erwähnt - nach Maniatis et al. 1982, Molecular Cloning: A Laboratory Manual, Cold Spring Harbor Laboratory und Ausubel et al. 1987, Current Protocols in Molecular Biology, John Wiley & Sons durchgeführt. Enzyme wurden von Gibco-BRL, Chemikalien von Sigma bezogen, Ausnahmen davon sind gekennzeichnet.

**Beispiel 1**

Klonierung und Charakterisierung des $\alpha$-$S_1$-Caseingenbereichs des Rindes

Der $\alpha$-$S_1$-Caseingenbereich des Rindes wurde über eine $\lambda$-Genbank isoliert. Aus Rinderlymphozyten (isoliert aus Rinderblut mit Ficoll Paque von Pharmacia LKB, gemäß den Angaben des Herstellers) isolierte DNA wurde mit dem Enzym Sau3AI (New England Biolabs) partiell gespalten. DNA-Fragmente im Größenbereich von 14 bis 21 Kb wurden über einen Salzgradienten angereichert, mit alkalischer Phosphatase (Boehringer Mannheim) behandelt und mit dem Vektor $\lambda$EMBL3 (Genofit, Heidelberg; Lit.: Frischauf et al. 1983, J. Mol. Biol. 170: 827-842) ligiert; wobei der Vektor zuvor mit den Enzymen BamHI und EcoRI gespalten worden war. Die ligierte DNA wurde in vitro mit einem $\lambda$-Verpackungsextrakt (Stratagene, Giga Pack Plus) gemäß den Angaben des Herstellers verpackt. Die so erhaltenen $\lambda$-Klone wurden auf dem E. coli-Stamm K803 vermehrt; aus $1\times10^6$ amplifizierten Klonen wurde eine Rindergenbank etabliert. $1\times10^6$ Klone der Genbank wurden parallel mit zwei Oligonukleotiden (CP4 und CP5) durchmustert. Die Oligonukleotide

**CP4: 5'- ATC ACC TTG ATC ATC AAC CCA GCT TGC TGC TTC TTT CCA GTC TTG GGT TC -3'**

**CP5: 5'- ATG AAA CTT TCT ATC CTT ACC TGT CTT GTG GCT GTT GCT CTT GCC -3'**

wurden gemäß den publizierten Sequenzen von Bonsing und Mackinlay 1987, Journal of Dairy Research 54: 447-461 (CP4: S. 451; CP5: S. 450) chemisch synthetisiert (Cyclone, Milligen-Biosearch). Sie entsprechen einem Teil des ersten, nichtkodierenden Exons (CP4) und dem für das Signalpeptid kodierenden Bereich (CP5). Positive Klone, die mit beiden Proben hybridisierten, wurden durch Subklonierungen und Spalten mit Restriktionsenzymen näher charakterisiert. Vom 5'- und vom 3'-Ende des Klons $\lambda$84 wurden jeweils repititionsfreie Fragmente (p26 bzw. p84, Abb. 1) isoliert. Mit ihnen wurde die Rindergenbank erneut durchmustert, positive Klone wurden wie beschrieben charakterisiert. Durch Sequenzanalyse von p19 und p86 (Abb. 1) konnte im Vergleich mit publizierten Sequenzen (Stewart et al. 1984, Nucl. Acids Res. 12: 3895-3907; Bonsing und Mackinlay 1987, Loc. cit., Yu-Lee et al. 1986, Nucl. Acids Res. 14: 1883-1902) gezeigt werden, daß sowohl das 5'- als auch das 3'-Ende des Rinder $\alpha$-$S_1$-Caseingens, sowie daran anschließende Genbereiche, isoliert worden waren. Damit waren insgesamt 40 Kb des $\alpha$-$S_1$-Caseingenbereichs (Abb. 1) isoliert worden.

**Beispiel 2**

Klonierung des Renningens des Rindes

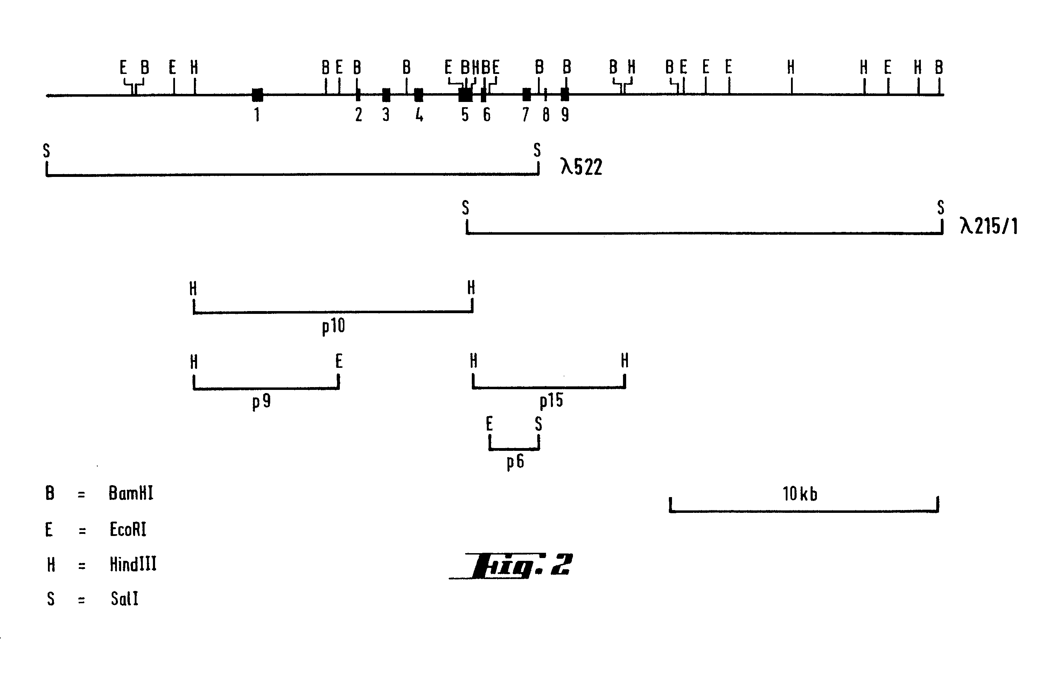

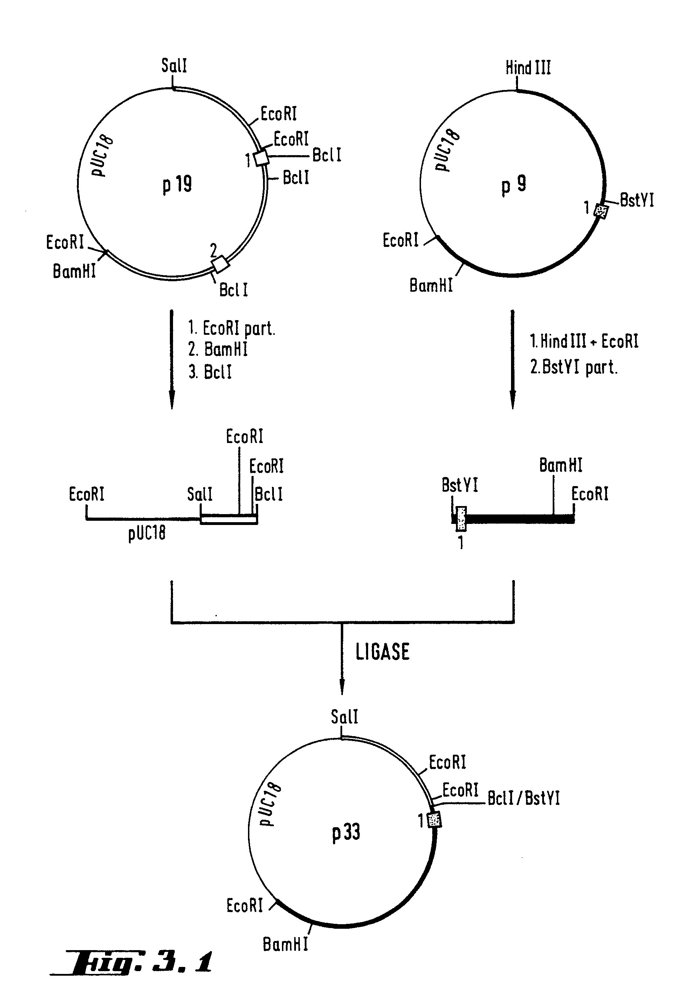

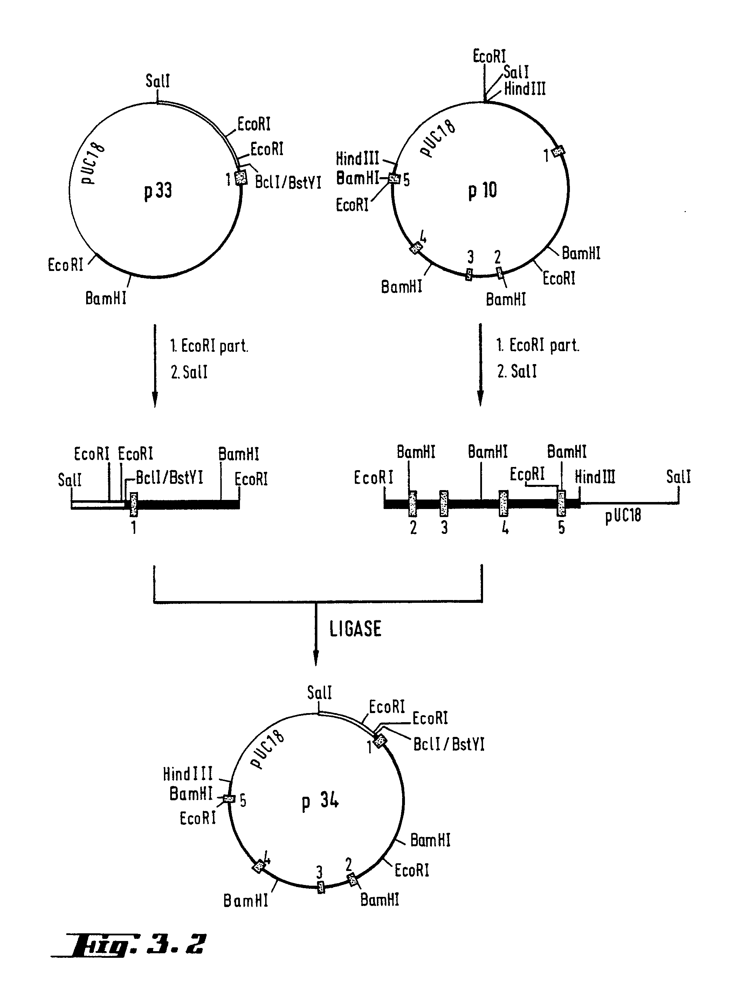

$1 \times 10^5$ Klone der unter Beispiel 1 beschriebenen Rindergenbank wurden mit dem Oligonukleotid RN(5'-GAG GTG TCT CGT GGT GCT ACT TGC TGT CTT CGC TCT CTC CCA GGG CAC -3') durchmustert. RN wurde chemisch (Cyclone, Milligen-Biosearch gemäß der publizierten Sequenz: Hidaka et al. 1986, Gene 43: 197-203) synthetisiert; das Oligonukleotid RN entspricht einem Teil des ersten Exons des Renningens (Position 28 - 75, Hidaka et al. 1986, loc. cit.). Von dem Klon λ522 (Abb. 2), der dabei erhalten worden war, wurde am 3'-Ende ein repititionsfreies DNA-Fragment isoliert (p6, Abb. 2), mit dem die Genbank erneut durchmustert wurde. Dabei konnte der Klon λ215/1 erhalten werden (Abb. 2). Beide Klone wurden durch Subklonierungen und Spalten mit Restriktionsenzymen charakterisiert. Durch Vergleich des Musters der Schnittstellen mit dem publizierten Bereich (Hidaka et al. 1986, loc. cit.) konnte gezeigt werden, daß beide Klone einen ca. 33 Kb großen Bereich des Renningens umfassen, das vollständig in diesem Bereich enthalten ist (Abb. 2).

**Beispiel 3**

Konstruktion von milchdrüsenspezifischen Expressionssystemen

a) Verknüpfung des bovinen $\alpha$-$S_1$-Caseinpromotors mit dem Renningen

Ein mögliches Protein, das durch den beschriebenen Prozeß in der Milch transgener Tiere gewonnen werden kann, ist Rennin. Die Konstruktion wird dabei so gewählt, daß Rennin in der inaktiven Form Prorennin gebildet wird, um eine Spaltung von Milchproteinen durch das aktive Rennin zu verhindern.

Das im E. coli-Stamm WA321 (AB1157, dam-4; Dreiseikelmann et al. 1979, Biochim Biophys Acta 562: 418-428) vermehrte Plasmid P19 (Abb. 1) wurde zunächst mit dem Enzym EcoRI partiell gespalten, anschließend wurde mit dem Enzym BamHI der Spaltansatz vollständig gespalten. Dadurch konnte das 6,7 Kb Fragment, das an der EcoRI-Schnittstelle im Polylinker gespalten worden war, von den anderen Spaltprodukten abgetrennt werden.

Dieses Fragment wurde mit BclI gespalten und das 4,2 Kb Spaltprodukt isoliert (Abb. 3.1), dies entspricht der Spaltung an Position 1485 (Anhang 1) des $\alpha$-$S_1$-Caseinpromotors. Die verwendete Spaltstelle liegt zwischen der TATA-box und dem Transkriptionsstart des $\alpha$-$S_1$-Caseingen, das Fragment enthält den unmittelbaren Promotorbereich.

Zur problemlosen Kombination des $\alpha$-$S_1$-Caseinpromotorbereiches mit dem Renningen eignet sich die BstYI-Schnittstelle, die zwischen der TATA-Box und dem Transkriptionsbeginn des Renningens liegt und die aufgrund gleicher überhängender Enden problemlos mit der BclI-Schnittstelle neu kombiniert werden kann. Dazu wurde das Konstrukt p9 (Abb. 2), das den Promotorbereich und das erste Exon des Rennins enthält, mit HindIII und EcoRI vollständig und anschließend mit BstYI partiell gespalten. Ein ca. 2,9 Kb großes Fragment wurde isoliert und mit dem BclI/EcoRI-Fragment ligiert (Abb. 3.1). Mit dem Ligationsansatz transformierte E. coli HB101 wurden untersucht, durch Sequenzanalyse konnte gezeigt werden, daß das Plasmid p33 (Abb. 3.1) auf dem 4,2 Kb insertierten Fragment die richtige Verknüpfung besaß.

Der Bereich von Exon 2 bis Exon 5 des Renningens wurden zu dem Konstrukt hinzugefügt, indem p33 mit EcoRI partiell und SalI vollständig gespalten (Abb. 3.2) und das 4,2 Kb Fragment isoliert wurde, das den $\alpha$-$S_1$-Caseingenpromotorbereich mit dem Exon 1 des Renningens enthielt. Dieses Fragment wurde mit einem 7,7 Kb Fragment ligiert (Abb. 3.2), das nach vollständiger Spaltung mit SalI und partieller Spaltung mit EcoRI aus p10 (Abb. 2) erhalten worden war (Abb. 3.2). Daraus wurde das Plasmid p34 (Abb. 3.2) erhalten, das den $\alpha$-$S_1$-Caseinpromotor sowie Exon 1 - 5 des Renningens enthielt.

Um das vollständige Renningen verknüpft mit dem $\alpha$-$S_1$-Caseinpromotor zu erhalten, wurde zunächst das Plasmid p34 mit HindIII linearisiert (Abb. 3.3) und mit alkalischer Phosphatase behandelt (Boehringer Mannheim). Aus dem Plasmid p15 (Abb. 2) wurde nach vollständiger Spaltung mit HindIII ein 5,5 Kb Fragment isoliert (Abb. 3.3), das Exon 6 - Exon 9 des Renningens enthielt. Das Fragment wurde mit dem linearisierten p34 ligiert, daraus konnte p42 (Abb. 3.3) erhalten werden, welches das komplette Renningen enthielt.

Bei Mikroinjektionsexperimenten zur Erzeugung transgener Tiere stören Plasmidanteile. Um das Expressionssystem einfach vom Vektoranteil trennen zu können, wurde p42 durch partielle Spaltung mit HindIII linearisiert und mit alkalischer Phosphatase (Boehringer Mannheim) behandelt. Die beiden phosphorylierten Oligonukleotide HS1 (5'- AGC TAG TCG ACT CTA GAC CGC GGT -3') und HS2 (5'- AGC TAC CGC GGT CTA GAG TCG ACT -3') wurden hybridisiert und mit dem linearisierten p42 ligiert. Durch Sequenzanalyse und SalI-Spaltung konnte gezeigt werden, daß p43 (Abb. 3.3) ein Oligonukleotidpaar an der richtigen Position enthielt; durch Spaltung mit SalI ist es möglich, das insertierte Fragment des p43 vom Vektoranteil zu trennen. Nach SalI-Spaltung von p43 konnte ein 14,7 Kb Fragment isoliert werden, welches das Renningen unter Kontrolle des $\alpha$-$S_1$-Caseinpromotors enthält. Signalsequenz und RNA-Terminationssignal stammen vom Renningen.

b) Verknüpfung des Renningens mit dem $\alpha$-$S_1$-Caseinpromotor über den für das Signalpeptid kodieren-den Bereich des $\alpha$-$S_1$-Caseingens

Nach Spaltung des Konstruktes p19 (Abb. 1) mit SacI und BglII wurde ein 5,6 Kb Fragment isoliert und mit dem Oligonukleotidpaar CIIR1/CIIR2 ligiert (Abb. 3.4). Das Fragment enthielt den Vektoranteil von p19 sowie den $\alpha$-$S_1$-Caseinpromotoranteil bis zur BglII-Schnittstelle an Position 2898 (Anhang 1), die kurz vor Beginn des zweiten Exons des $\alpha$-$S_1$-Caseingens liegt (Position 2900, Anhang 1). Die Oligonukleotide CIIR1 und CIIR2 wurden chemisch synthetisiert (Cyclone, Milligen-Biosearch), phosphoryliert und miteinander hybridisiert. Sie enthielten die kodierende Information für das Signalpeptid des bovinen $\alpha$-$S_1$-Caseingens verknüpft mit den Aminosäuren 1 bis 6 des bovinen Prorennins, sowie aus klonierungs-technischen Gründen weitere Sequenzen:

```
                        BglII
                              ┌──────────────────────→
   CIIR1    5'- GAT CTT GAC AAC CAT GAA ACT TCT -
   CIIR2        3'- AA CTG TTG GTA CTT TGA AGA -

           α-S₁-Casein-Signalpeptid
                              ←──────────────────────
         - CAT CCT TAC CTG TCT TGT GGC TGT -
         - GTA GGA ATG GAC AGA ACA CCG ACA -

                                   Prorennin
            ←──────────────┐ ┌──────────────→
         - TGC TCT TGC CAC TGA GAT CAC CAG -
         - ACG AGA ACG GTG ACT CTA GTG GTC -
                        SacI

            ←──────┐
         - GAT CCG AGCT -3'
         - CTA GGC -5'


                        BamHI
```

Durch Sequenzierung wurde bestätigt, daß der Klon p73 (Abb. 3.4) die Konstruktion in der richtigen Anordnung enthielt. Die BamHI-Schnittstelle im Oligonukleotidpaar CIIR1/CIIR2 ermöglichte eine Fusion an das zweite Exon des Renningens an der Aminosäure 6 des Prorennins, wobei der Leserahmen erhalten blieb. Dazu wurde zunächst das Plasmid P10 (Abb. 2), das Exon 1 - 5 des Renningens enthielt,

9

mit SalI vollständig und BamHI partiell gespalten (Abb. 3.5). Ein 6,8 Kb Fragment, das den Vektoranteil von p10 sowie Exon 2 - 5 des Renningens enthielt, wurde mit einem 3 Kb Fragment ligiert, welches nach Spaltung von p73 (Abb. 3.4) mit den Enzymen BamHI und SalI erhalten worden war (Abb. 3.5).

Der Klon p75 (Abb. 3.5) enthält Exon 2 - Exon 5 des Renningens mit dem α-S$_1$-Caseinpromotor über das Signalpeptid α-S$_1$-Caseins verknüpft. Der Vektoranteil wurde von p75 nach Spalten mit SalI und HindIII entfernt (Abb. 3.6), das 7,1 Kb Fragment wurde mit einem 7,3 Kb Fragment ligiert, welches nach Spaltung von p43 (Abb. 3.3) mit HindIII (vollständig) und SalI (partiell) erhalten worden war (Abb. 3.6) und Exon 6-Exon 9 des Renningens enthält. Der daraus resultierende Klon p77 (Abb. 3.6) enthält die genetische Information für das Prorennin, welches mit der für das Signalpeptid des α-S$_1$-Caseins kodierenden Sequenz verknüpft war. Daneben enthält p77 den α-S$_1$-Caseinpromotor und das erste nichtkodierende Exon sowie das erste Intron des α-S$_1$-Caseingens verknüpft mit der α-S$_1$-Casein-Signalpeptidsequenz in der natürlichen Anordnung. Terminationssignal und Polyadenylierungsstelle stammen vom Renningen. Durch Spaltung mit SalI konnte das Expressionssystem vom Vektoranteil getrennt werden.

c) Verknüpfung des Renningens mit dem α-S$_1$-Caseinpromotor, dem α-S$_1$-Signalpeptid sowie 3'-nichttranslatierten Sequenzen des α-S$_1$-Caseingens

Das Konstrukt p86 (Abb. 1) wurde mit BamHI und SalI gespalten und mit dem Oligonukleotidpaar BSI/BSII (BSI: 5'- GAT CTG GGG TCT CCT ACC ATC G -3'; BSII: 5'- TCG ACG ATG GTA GGA GAC CCC A -3') ligiert (Abb. 3.7). BSI und BSII waren chemisch synthetisiert worden (Cyclone, Millingen-Biosearch). Damit wurde die BamHI-Schnittstelle entfernt (≙ p87; Abb. 3.7). Das Konstrukt p87 wurde mit EcoRI linearisiert und mit dem Oligonukleotidpaar C3'RI/C3'RII ligiert (Abb. 3.7). Die Oligonukleotide C3'RI und C3'RII:

```
C3'RI    5'- AAT TGT CGA CGG ATC CTG GGG -

C3'RII       3'-CA GCT GCC TAG GAC CCC -


C3'RI      - GAT GTT TTC ATC CGA GAG TAT -

C3'RII     - CTA CAA AAG TAG GCT CTC ATA -


C3'RI      - TAC AGC GTC TTT GAC AGG GCC -

C3'RII     - ATG TCG CAG AAA CTG TCC CGG -


C3'RI      - AAC AAC CTC GTG GGG CTG GCC -

C3'RII     - TTG TTG GAG CAC CCC GAC CGG -


C3'RI      - AAA GCC ATC TGA GGA GTC AAG -

C3'RII     - TTT CGG TAG ACT CCT CAG TTC -


C3'RI      - TG -3'

C3'RII     - ACT TAA -5'
```

waren chemisch synthetisiert worden (Cyclone, Milligen-Biosearch) und enthielten den kodierenden Teil des neunten Exons 3'-seitig von der BamHI-Schnittstelle bis zum Stopkodon, daran anschließend den 3'-nichtkodierenden Bereich nach dem Stopkodon des α-S$_1$-Caseingens bis zur EcoRI-Schnittstelle (Position 724, Stewart et al. 1984, Nucl. Acids Res. 12: 3895-3907). Durch Sequenzierung wurde bestätigt, daß der Klon p94 das Oligonukleotidpaar in der richtigen Orientierung enthält. Das Konstrukt p94 wurde mit SalI partiell und BamHI vollständig gespalten (Abb. 3.8). Ein 6,2 Kb Fragment enthielt den Vektoranteil sowie das vollständige Insert. Dieses Fragment wurde mit einem 10,7 Kb Fragment ligiert (Abb. 3.8), das nach Spaltung von p77 (Abb. 3.6) mit SaII (vollständig) und BamHI (partiell) erhalten wurde (Abb. 3.8). Dieses Fragment enthielt das vollständige Expressionssystem des p77 mit Ausnahme des 3'-Anteils nach der BamHI-Schnittstelle im neunten Exon des Renningens. Das Konstrukt p99 (Abb. 3.8) enthielt nun ein Expressionssystem, das dem von p77 (s. Beispiel 3b) entsprach, bei dem jedoch der 3'-Bereich des Renningens unmittelbar nach dem Stopkodon ersetzt durch den unmittelbar an das Stopkodon des $\alpha$-S$_1$-Caseingens anschließenden DNA-Bereich worden war. Dieser Bereich enthält neben einem Intron ein Exon, das die Signale für die Termination der Transkription und für die Polyadenylierung enthielt. Auf diese Weise wurde eine Expressionskassette konstruiert, die 5'- und 3'-regulierende Sequenzen aus dem $\alpha$-S$_1$-Caseingenbereich sowie für Prorennin kodierende Sequenzen enthält. Sie kann durch SaII-Spaltung des Plasmids p99 vom Vektoranteil getrennt werden.

d) Expressionssystem für menschlichen IGF-I unter Kontrolle 5,- und 3'-regulierender DNA-Sequenzen aus dem bovinen $\alpha$-S$_1$-Caseingenbereich

Das Konstrukt p19 (Abb. 1) wurde mit EcoRI partiell und BgIII vollständig gespalten (Abb. 3.9). Ein 5,6 Kb Fragment wurde isoliert, das den $\alpha$-S$_1$-Caseinpromotorbereich, das erste nichtkodierende Exon sowie Intron A bis Position 2898 (Anhang 1) enthielt. Mit diesem Fragment wurde ein anderes DNA-Fragment ligiert, das aus den. 6 Oligonukleotiden IF1 - IF6 bestand, die chemisch synthetisiert worden waren (Cyclone, Milligen-Biosearch).

Dazu waren zunächst die Oligonukleotide IF1 und IF2, IF3 und IF4 sowie IF5 und IF6 getrennt hybridisiert worden. Die Hybridisierungsansätze wurden zusammengegeben und ligiert (Abb. 3.10 a), bevor sie mit dem aus p19 isolierten Fragment (s.o.) ligiert wurden (Abb. 3.9). Die Oligonukleotide IF1 - IF6 enthalten die Information ab Position 2899 (Anhang 1) des bovinen $\alpha$-S$_1$-Caseingens bis zum Ende des für das Signalpeptid kodierenden Bereichs (Position 2956). Direkt daran schließt im Leserahmen ein DNA-Bereich an, der die Information für das reife menschliche IGF-1 trägt (Aminosäuresequenz: Tavekkol et al. 1988, Mol. Endo. 2: 648-681). Die Nukleinsäure-Kodons wurden nach der Benutzungshäufigkeit im Kaninchen ausgewählt (nach einer Methode von R. Lathe 1985, J. Mol. Biol. 183: 1-12). An diesen Bereich anschließend folgt ein Stopkodon, der 3'-nichttranslatierte Bereich des $\alpha$-S$_1$-Caseingens (siehe Beispiel 3c) bis zur EcoRI-Schnittstelle an Position 724 (Stewart et al. 1984, Nucl. Acids Res. 12: 3895-3907) sowie weitere, für den Klonierungsvorgang wichtige Sequenzen (Abb. 3.10b).

Durch Sequenzierung wurde bestätigt, daß der Klon p98 (Abb. 3.9) die korrekte Information enthielt. Das Konstrukt p98 wurde mit EcoRI partiell und BamHI vollständig gespalten. Das isolierte 5,9 Kb Fragment, das den Vektoranteil sowie das Insert von p98 enthielt, wurde mit dem 3,5 Kb EcoRI/BamHI Fragment aus p86 (Abb. 1) ligiert (Abb. 3.9).

Das Konstrukt p100 wurde durch Restriktionsschnitt charakterisiert, es enthielt die IGF-I Expressionskassette in der korrekten Orientierung. Die IGF-I-Expressionskassette benutzt zur Steigerung der Expression die Spleißstellen des ersten und des letzten Introns des bovinen $\alpha$-S$_1$-Caseingens sowie diese Introns selbst. In der Expressionskassette ist die für das reife IGF-I kodierende Sequenz über die Signalsequenz des $\alpha$-S$_1$-Caseins mit dem $\alpha$-S$_1$-Caseinpromotorbereich und dem ersten Intron des $\alpha$-S$_1$-Caseingens verbunden. Im Anschluß an das Stopkodon folgt eine 3,5 Kb 3'-Sequenz des $\alpha$-S$_1$-Caseingens, welche die DNA-Sequenz ab dem Stopkodon des $\alpha$-S$_1$-Caseingens, das letzte Intron sowie das letzte, nichttranslatierte Exon des bovinen $\alpha$-S$_1$-Caseingens und weitere nichttranslatierte 3'-Sequenzen enthält. In diesem Exon sind die Signale für die Polyadenylierung enthalten. Die Expressionskassette kann vom Vektoranteil nach SaII-Spaltung abgetrennt werden.

e) Entwicklung einer Expressionskassette zur Synthese von Proteinen in der Milchdrüse transgener Tiere

Das Konstrukt p100 (Abb. 3.9) wurde mit EcoRI partiell und mit BgIIII vollständig gespalten (Abb. 3.11). Das linearisierte 9,1 Kb große Fragment wurde mit dem hybridisierten Oligonukleotidpaar BgEI/BgEII ligiert. Die zueinander komplementären Oligonukleotide BgEI (5'- GAT CTA GAC TAG GGT AAG GGT TGC TG -3') und BgEII (5'- AAT TCA GCA ACC CTT ACC CTA GTC TA -3') waren chemisch synthetisiert worden (Cyclone, Milligen-Biosearch). Das daraus resultierende Konstrukt p115 (Abb. 3.11) enthielt das Oligonukleotidpaar. Durch partielle Spaltung von p115 mit EcoRI und nachfolgender Spaltung mit BgIII konnte ein 9,1 Kb Fagment isoliert werden, das 5,- und 3'-Anteile des $\alpha$-S$_1$-Caseingenbereichs enthielt. Über die BgIII- und EcoRI-Schnittstellen können beliebige, für Proteine kodierende DNA-Fragmente mit dem aus p115 isolierten Fragment verknüpft werden und zur Expression in der Milchdrüse transgener Tiere verwendet werden (s. nachfolgendes Beispiel).

f) Expressionssystem für Rennin unter Kontrolle regulierender 5'- und 3'-Sequenzen aus dem bovinen $\alpha$-S$_1$-Caseingenbereich unter Verwendung eines synthetischen Renningens

Aus 26 Oligonukleotiden wurde ein synthetisches Renningen gebildet, das die Information für die Bildung des Prorennin verknüpft mit dem Signalpeptid bovinen $\alpha$-S$_1$-Caseins enthält. Die DNA-Sequenz des Prorennins wurde gemäß der Aminosäuresequenz (Hidaka et al. 1986, Loc. cit.) gewählt.

11

Vorzugsweise wurden die natürlichen Kodons verwendet, aus klonierungstechnischen Gründen wurden jedoch Enzymschnittstellen hinzugefügt oder deletiert. Folgende Oligonukleotide wurden nach der Sequenz des kodierenden Stranges (Anhang 2a) gebildet:

| Nummer | Position 5' - 3' | |
|---|---|---|
| R01 | 5 - 99 | |
| R03 | 100 - 210 | |
| R05 | 211 - 315 | |
| R07 | 316 - 416 | |
| R09 | 417 - 518 | + 5'- GGG AGCT -3' |
| R11 | 519 - 609 | |
| R13 | 610 - 699 | |
| R15 | 700 - 789 | |
| R17 | 790 - 872 | |
| R19 | 873 - 949 | |
| R21 | 950 - 1034 | |
| R23 | 1035 - 1119 | |
| R25 | 1120 - 1209 | |

Nach der Sequenz des dazu komplementären Stranges (Anhang 2b) wurden die folgenden Oligonukleotide synthetisiert:

| Nummer | Position 5' - 3' |
|---|---|
| R02 | 1100 - 1210 |
| R04 | 989 - 1099 |
| R06 | 900 - 988 |
| R08 | 807 - 899 |
| R10 | 690 - 806 |
| R12 | 614 - 692 |
| R14 | 524 - 613 |
| R16 | 434 - 523 |
| R18 | 339 - 433 |
| R20 | 250 - 338 |
| R22 | 165 - 249 |
| R24 | 80 - 164 |
| R26 | 6 - 79 |

Das gesamte Renninkonstrukt wurde aus 4 Blöcken zusammengesetzt. Jeder Block bestand aus mehreren Paaren zueinander komplementärer Oligonukleotide (z.B. Block II:R07/R08 und R09/R10), die zunächst in getrennten Ansätzen hybridisiert wurden, bevor die Oligonukleotidpaare eines Blockes miteinander ligiert und kloniert wurden. Die Blöcke bestanden aus den folgenden Oligonukleotiden (Abb. 3.12): Block I: R01 - R06; Block II: R07 - R10; Block III: R11 - R18 und Block IV: R19 - R26.

Der Klonierungsvorgang ist schematisch in Abb. 3.13 dargestellt, sämtliche Konstrukte wurden durch Sequenzierung überprüft. Block I wurde über PstI- und KpnI-Schnittstellen in den Vektor pUC18 kloniert. Das daraus resultierende Konstrukt p110 wurde mit KpnI und SacI linearisiert und mit Block II ligiert. Das resultierende Konstrukt p111 enthielt die Blöcke I und II ($\triangleq$ R01 - R10). Block IV wurde in einen mit EcoRV und KpnI linearisierten Bluescript-Vektor (Fa. Stratagene) kloniert. Das daraus resultierende Konstrukt p112 wurde mit SmaI und EcoRV linearisiert und mit Block III ligiert. Das Konstrukt p113 enthielt die Blöcke III und IV ($\triangleq$ R11 - R26). Das resultierende Konstrukt p111 wurde mit SmaI und SacI linearisiert und mit einem Fragment ligiert, das nach Spaltung von p113 mit SmaI und SacI erhalten wurde und den ligierten Blöcken III und IV entsprach. Das Konstrukt p114 enthielt die gesamte, für das $\alpha$-S$_1$-Caseinsignalpeptid und Prorennin kodierende genetische Information.

Um diese genetische Konstruktion in die Expressionskassette P115 einzusetzen, wurde p115 mit EcoRI partiell und mit BglII vollständig gespalten. (Abb. 3.14). Das linearisierte 9,1 Kb große Fragment wurde mit dem 1,2 Kb Fragment ligiert, das nach Spaltung des Konstruktes p114 mit den Enzymen BglII und EcoRI erhalten wurde und die genetische Information für das Prorennin verknüpft mit dem $\alpha$-S$_1$-Caseinsignalpeptid enthielt. Das Konstrukt p116 (Abb. 3.14) enthielt das synthetische Renningen verknüpft mit dem $\alpha$-S$_1$-Signalpeptid unter Kontrolle 5'- und 3'-regulierender Sequenzen aus dem bovinen $\alpha$-S$_1$-Caseingenbereich. Nach Spaltung mit SalI konnte das Expressionssystem vom Vektoranteil getrennt werden.

## Beispiel 4

Erstellung transgener Säugetiere

Die Erstellung transgener Säugetiere umfaßt die Herstellung injizierbarer DNA-Lösung, die Gewinnung befruchteter Eizellen und Embryonen, die Mikroinjektion der DNA-Lösung in Vorkerne oder Kerne, den Transfer der injizierten Zygoten auf synchronisierte Empfängertiere und die Untersuchung der geborenen Tiere auf Integration. Dabei sind für die einzelnen Säugerspezies wie Kaninchen oder Rind einige speziesbedingte Unterschiede bei der Vorbereitung der Spender- und Empfängertiere, der Gewinnung und dem Transfer der Embryonen sowie der Mikroinjektion zu beachten.

a) Herstellung einer injizierbaren DNA-Lösung

Von Vektormolekülen abgetrennte DNA-Fragmente (über Gelelektrophorese), welche die vollständigen Expressionskonstrukte enthielten, wurden in steril filtriertem TE-Puffer (10 mM Tris, 0,2 mM EDTA, pH 7,6) zu einer Konzentration von 1000 Kopien pro Picoliter aufgenommen und für die Mikroinjektion verwendet.

b) Gewinnung von Embryonen aus Kaninchen

Geschlechtsreife Spenderkaninchen erhalten zur Superovulation eine einmalige Gabe von 10 bis 40 IE PMSG (Pregnant Mares' Serum Gonadotropin) pro kg Körpergewicht. Dieser Superovulation ging eine 21tägige Einzelhaltung oder eine Vorsynchronisation mit 120 IE HCG (Human Chorionic Gonadotropin) voraus. 72 bis 76 Stunden nach der PMSG-Injektion werden die Kaninchen zweimal im Abstand von einer Stunde künstlich besamt oder im Natursprung belegt. Unmittelbar danach erhalten sie zur Ovulationsinduktion 120 bis 180 IE HCG i.v.. Die Embryogewinnung erfolgt 19 bis 21 Stunden nach der Belegung durch Schlachttötung der Spenderkaninchen. Die Eileiter werden mit Kulturmedium für Kaninchenembryonen (BSM II + 20% FKS oder PBS + 20% FKS) durch Spülung vom Fimbrientrichter in Richtung Uterushornende gewonnen. Falls erforderlich, wird der noch vorhandene Cumulus Oophorus durch Hyaluronidase-Behandlung entfernt. Die Embryonen werden gewaschen und bis zur Mikroinjektion kultiviert.

c) Gewinnung von Embryonen aus Rindern

Rinder und Kühe werden nach gynäkologischer Untersuchung unabhängig vom Zyklusstand durch zweimalige PG-Applikation (PG-F2 $\alpha$- Analog Estrumate, 500 mg Cloprostenol-Natriumsalz, i.m.) im Abstand von 12 Tagen synchronisiert. 48 bis 72 Stunden später sind die Tiere dann im Brunst. 11 bis 14 Tage nach dieser Brunst erhalten die Tiere morgens um 6 Uhr 2000 bis 3000 I.E. PMSG i.m. und nach 60 Stunden eine weitere PG-Applikation. Zwei Tage später kommen die Tiere in Brunst und werden um 18.00 Uhr besamt. Am darauffolgenden Tag um 6.00 Uhr erfolgt die zweite Besamung. Einen Tag später

13

erfolgt zwischen 7.00 und 9.00 Uhr nach Schlachtung der Spendertiere die Gewinnung der Genitalorgane. Dazu wird nach dem Betäubungsschuß während des Ausblutens kranial des Euters ein etwa zwei handbreiter Schnitt in der Linie alba gesetzt und die Bauchhöhle geöffnet. Zervix und Gebärmutter mit Eileitern und Eierstöcken werden entnommen. Die Eileiter werden von den Eierstöcken freipräpariert und am uterotubalen Übergang abgetrennt. Eine 2,5 mm dicke Knopfkanüle wird in das Infundibulum eingeführt und fixiert. Der Eileiter wird mit etwa 20 ml PBS (Phosphat Buffer Saline) mit 20 % FCS (Fetal Calf Serum) durchgespült. Die in einer Petrischale aufgefangene Spülflüssigkeit wird unter dem Mikroskop nach Eizellen abgesucht. Die gefundenen Eizellen werden gewaschen, in Embryogläser verpackt und ins Labor transportiert.

d) Mikroinjektion der DNA-Lösung

Für die Mikroinjektion werden ein Inversmikroskop (Zeiss, ICM 405) zwei Leitz-Mikromanipulatoren und ein Injektionsgerät (Eppendorf) verwendet. Der eine Manipulator trägt eine Haltepipette, mit der der Embryo durch Unterdruck fixiert werden kann. Auf dem zweiten Mikromanipulator wird die mit DNA-Lösung gefüllte Injektionspipette in einem Nanostepper fixiert und mit dem Injektionsgerät verbunden. Die Spitze der Injektionspipette hat einen Durchmesser von 1 bis 2 $\mu$m. Zur Mikroinjektion wird die Pipettenspitze durch die Zona Pellucida, die Zellmembran und die Kernmembran in das Kernlumen vorgeschoben und ca. 1 bis 2 pl DNA-Lösung werden dort abgesetzt. Die Volumenzunahme des Vorkerns signalisiert eine erfolgreiche Mikroinjektion. Z. T. erfolgt auch eine Mikroinjektion der Kerne von Embryonen im Zweizellstadium. Die Mikroinjektion erfolgt in einem Mediumtropfen auf einem Deckglas oder in einer sogenannten Injektionskammer. Nach der Mikroinjektion werden die Eizellen oder Embryonen bis zum Transfer kultiviert.

In Rindereizellen und in -embryonen sind aufgrund der dunklen lipidhaltigen Granula keinerlei Kernstrukturen erkennbar. Durch Zentrifugation der Eizellen bzw. Embryonen in einer Eppendorf-Zentrifuge bei 15.000 g für ca. 3 Minuten können die Kerne sichtbar gemacht werden.

e) Kultur und Transfer der mikroinjizierten Eizellen und Embryonen bei Kaninchen

Empfängerkaninchen werden 21 Tage vor dem voraussichtlichen Termin zur Zyklussynchronisation einzeln aufgestallt und erhalten zur Induktion einer Pseudogravidität 120 IE HCG. Am Tag vor dem Transfer erhalten die Empfängerkaninchen zur Ovulationsinduktion 120 IE HCG i.v. Zum Transfer werden die Kaninchen mit 0,4 ml Rompun 2 % pro kg/Körpergewicht und 0,8 ml Ketamin 10% pro kg/Körpergewicht narkotisiert. Nach Vorbereitung des Operationsfeldes und Entleerung der Harnblase durch transabdominalen Druck werden die Kaninchen in Rückenlage ausgebunden und in Schräglage fixiert. Unmittelbar kaudal des Nabels wird die Bauchhöhle in einer Länge von ca. 4 bis 5 cm geöffnet. Ovar, Eileiter und kraniales Uterushorn werden vorgelagert und nach Kontrolle der Ovarreaktion werden die mikroinjizierten Embryonen mit Hilfe einer Transferpipette ca. 2 bis 2,50 cm tief in die Eileiterampulle transferiert. Pro Seite werden zwischen 8 und 15 Embryonen transferiert. Nach Verschluß der Bauchhöhle wird die Operationswunde durch eine Hautfaltendecknaht geschützt. Die Geburt der Jungtiere erfolgt 29 bis 31 Tage nach dem Transfer.

f) Kultur und Transfer der mikroinjizierten Eizellen und Embryonen bei Rindern

- in vitro Kultur

Die Kultur der mikroinjizierten Zygoten wird in vitro durchgeführt. Für diese in vitro Kultur werden Granulosazellen aus dem Kumulus oder Epithelzellen aus dem Eileiter mit den Embryonen kokultiviert. Als Medium verwenden wir das modifizierte Parker Medium TCM 199. Von entscheidender Bedeutung ist, daß die Kultur in einer Gasatmosphäre mit reduziertem Sauerstoffgehalt (5 % $O_2$) und bei 5 % $CO_2$ in 90% Stickstoff erfolgt. Die Kultur wird bei 39°C und maximaler Luftfeuchtigkeit durchgeführt. In 6 Tagen entwickeln sich die injizierten Eizellen in der in vitro Kultur bis zu Morula/-Blastozystenstadien, die für den unblutigen Transfer geeignet sind.

- Transfer auf Empfängertiere

Die Empfängertiere erhalten gleichzeitig mit den Spendertieren am Tag 2 eine PGF2-Injektion zur Zyklussynchronisation. Eine Sedierung der Empfängertiere (6,8 mg Acepromazinmaleat pro 100 kg Körpergewicht) und eine kleine Epiduralanästhesie (Lidocain 2%ig) erleichtert die Durchführung der Embryoübertragung. Der unblutige Transfer wird mit Hilfe eines sterilen Transfergerätes (Firma Wörrlein) vorgenommen, wobei der Embryo möglichst tief in das ipsilaterale Uterushorn übertragen wird. Nach dem Transfer erhalten die Empfänger Clenbuterolhydrochlorid als Uterusrelaxans. Fünf bis sechs Wochen nach dem Transfer können die Empfängertiere durch rektale Palpation auf Trächtigkeit untersucht werden.


**Beispiel 5**

Untersuchung der Integration von Konstrukten im Kaninchengenom nach Mikroinjektionsexperimenten

Von Kaninchen, die z.B. mit dem Konstrukt p77 (Beispiel 3b) gemäß Beispiel 4 erzeugt worden waren, wurden zur Untersuchung Gewebeproben (meist Schwanz) verwendet. 20 μg der aus dem Gewebe isolierten DNA wurden mit 100 U EcoRI gespalten und zusammen mit einer DNA-Probe eines negativen Kaninchens einer Gelelektrophorese unterzogen. Die über ein Agarosegel aufgetrennten Fragmente wurden mit einer Vacu-Blot-Apparatur (Pharmacia - LKB) über Nitrocellulosefilter (Schleicher + Schuell, BA85) übertragen und mit radioaktiv markierten p26 (Abb. 1) hybridisiert. Das transgene Kaninchen # 2848 konnte dadurch leicht identifiziert werden

**Beispiel 6**

Untersuchung der Integration von Konstrukten im Rindergenom nach Mikroinjektionsexperimenten

Die Untersuchung wurde analog Beispiel 5 durchgeführt, jedoch wurde zur Hybridisierung der Filter eine radioaktive Probe verwendet, die frei von Repititionen war und aus dem $\alpha$-$S_1$-Caseinpromotor entnommen war ($\triangleq$ p26, Abb. 1). Durch Analyse der Fragmentgrößen konnte das endogene hybridisierende Fragment von dem des Transgens unterschieden werden.

**Beispiel 7**

Analyse der Nachkommen transgener Tiere

Die Analyse wurde durch Southern-Blot-Experimente wie unter Beispiel 5 durchgeführt. Zur Kontrolle wurde auch DNA aus negativen Kaninchen und dem transgenen Elterntier (# 2848, s. Beispiel 5) untersucht. Anhand des Restriktionsspaltmusters wären etwaige genetische Umlagerungen zu erkennen gewesen, die jedoch nicht auftraten.

**Beispiel 8**

Untersuchung der Milch transgener laktierender Kaninchen, die mit Konstrukt p43 erzeugt wurden

a) Rennin-Aktivitätstest
0,5 ml Milch eines transgenen laktierenden Kaninchens, das mit p43 (siehe Beispiel 3a) erzeugt worden war, wurden mit 0,5 ml Ca-Acetat-Puffer (10 mM $CaCl_2$, 100 mM NaAc, pH 6) und 20 Minuten bei 4°C und 3000 g zentrifugiert. Anschließend wurde die Fettphase abpipettiert und verworfen. Die restliche Suspension (ca. 0,8 ml) wurde mit HCl auf pH 2,5 eingestellt und die ausgefallenen Proteine abzentrifugiert (RT, 5 min, 13000 g). Die autokatalytische Aktivierung des Rennins wurde 90 Minuten bei 30°C durchgeführt. Anschließend wurde mit NaOH der pH-Wert auf 6,5 eingestellt. Der erneut entstandene Niederschlag wurde abzentrifugiert (RT, 5 min, 13000 g) und verworfen. 0,5 ml der klaren Lösung wurden mit 0,5 ml käuflicher Vollmilch (pasteurisiert) vermischt und bei 30°C inkubiert und die Zeit bis zum Beginn der Gerinnung bestimmt.
So behandelte Milch transgener Kaninchen zeigte Gerinnungsaktivität im Gegensatz zu ebenso behandelter Milch negativer Kontrolltiere.
b) Immunnachweis von Prorennin und Rennin in der Milch transgener Tiere, welche mit dem Konstrukt p43 erzeugt wurden
Nach a) behandelte Milchproben sowie unbehandelte Proben, denen das Fett entnommen worden war (4°C, 10 min, 3000 g), wurden auf denaturierenden PAA-Gelen aufgetrennt. (s. Ausubel et al. 1987, Current Protocols in Molekular Biology, John Wiley & Sons, Punkt 10.2.1). Die Proteine wurden anschließend mit einem Elektroblotgerät (Pharmacia - LKB) auf Nitrocellulosefilter (BA85, Schleicher + Schuell) übertragen. Transferiertes Prorennin und Rennin wurden mit polyklonalen Antikörpern detektiert, die nach Immunisierung von Kaninchen mit Rennin (Sigma) gewonnen worden waren. Als Detektionssystem wurde an Protein A gekoppelte Horse-radish Peroxidase (Amersham) verwendet. (Transfer und Immunodetektion: Ausubel et al. 1987, loc cit, Punkte 10.8.1 - 10.8.4).
Transgene Tiere enthielten 0,2 bis 0,6 mg/ml Prorennin in ihrer Milch. Laktierende Nachkommen, die ebenfalls das Konstrukt p43 in ihrem Genom enthielten, produzierten vergleichbare Mengen Prorennin.

**Beispiel 9**

Untersuchung der Milch transgener laktierender Kaninchen, die mit Konstrukt p77 (Beispiel 3 b) erzeugt wurden.

a) Rennin-Aktivitätstest

Der Test wurde analog Beispiel 8 a) durchgeführt. Eine Gerinnungsaktivität konnte ebenfalls nur mit der Milch transgener Tiere erzielt werden.

b) Immunnachweis von Prorennin und Rennin in der Milch transgener Tiere, welche mit dem Konstrukt p77 erzeugt wurden

Der Nachweis verlief analog Beispiel 8 b).

Transgene Tiere enthielten 1 bis 4 mg/ml Prorennin in ihrer Milch. Laktierende Nachkommen, die ebenfalls das Konstrukt p77 in ihrem Genom enthielten, produzierten vergleichbare Mengen Prorennin.

Anhang 1

```
              10         20         30         40         50
5' GATCTTCCCA ATCCAGATAT TGAACCTGCA TCTCTAATGT TTCCTGCATT
              60         70         80         90        100
   GGCAGGCAGG TTCTTTACCA CTAGTGCCAC CTGGAAAGTC CGGATACACT
             110        120        130        140        150
   CCTGGGAAAG ACAAAAGTAG AGTATTACAA TGCAGCAAGG ATTTTTGTTC
             160        170        180        190        200
   TCAGCTCCTT GAATAAATTA TAGTGAATAG AAAACATTAG TATCTTGTTG
             210        220        230        240        250
   AAATTGATGT GAAACAGATA GTAAGGAAGA TAATATCTAA AGAAAACTTC
             260        270        280        290        300
   AATATGGGAA ATTATAGTCT TTTCTATCTT CAAAGTGGAC AGCCTGAACA
             210        320        330        340        350
   GTTTTGAAAT TTCTTTTAAT ACAAAATAAT GTTCCTGTCA TACAACTGTG
             360        370        380        390        400
   AATACACTGA AAATATCACT ATAGATTTTT TAAAGTATAT AATATGATTC
             410        420        430        440        450
   CTTTCTTATA AACAATGAGT TGCAATCAAC AAGTTTTTAA AGCTCTCACT
             460        470        480        490        500
   TGTATAGATT TATTTTTAGC ACATAATATT TTTCTACAAT GTACAATTCC
             510        520        530        540        550
   AGTTAATTCT AGGAGTACAA TTAAGAATTG GAGAGATAGG AATTTTTTTC
             560        570        580        590        600
   TTTTACTTGT TTACTTTAAA AGATGGAAAA TCAGAGTTAT GGTTTATTTT
             610        620        630        640        650
   TCGCAATATT TAAAAATTAT AATTCTTGAA TAACTATTAA TTTTAATTAA
             660        670        680        690        700
   ATAATCTGTA ATGAGAATCC TCCTACCAAT GTAGGAGACG TGAGTTTGAC
             710        720        730        740        750
   TCCCGGGTAG GGAAGATACC CTGCAGAAGG AAATGGCAAC CCACTCCAAT
             760        770        780        790        800
   ATTATTACTT GGGAAATCCC ATGGACAGAG GAGACTGGCA GGCTGCAGTC
             810        820        830        840        850
   CATGGGGGTC ACAAAGAACT GGACACGACT TAGAAACTAA ACAACAACAA
             860        870        880        890        900
   TTTATACCAG AATGAATGAA CTAGTTACCA CAACTAGTAC ACCCAAAATG
             910        920        930        940        950
   AACAAAAAAT AGCTTGGTGG TATAATTAAA ATGCCACCAA AATTTATACA
             960        970        980        990       1000
   ATAATTATAT TTTCTTTTTG CAGGAAAAAG ATTAGACCAC ATATAATGTA
            1010       1020       1030       1040       1050
   ACTTATTTCA CAAGGTAAAT AATTATAATA AATAATATGG ATTAACTGAG
            1060       1070       1080       1090       1100
   TTTTAAAAGG TGAAATAAAT AATGAATTCT CTCATGGTC TTGTATGTTA
            1110       1120       1130       1140       1150
   ATAAAAATTG AAAAATTTTG AAGACCCCAT TTTGTCCCAA GAATTTCATT
            1160       1170       1180       1190       1200
   TACAGGTATT GAATTTTTCA AAGGTTACAA AGGAAATTTT ATTGATATAA
            1210       1220       1230       1240       1250
   TAAATGCATG TTCTCATAAT AACCATAAAT CTAGGGTTTT GTTGGGGTTT
            1260       1270       1280       1290       1300
   TTTTTGTTTG TTAATTTAGA ACAATGCCAT TCCATTTCCT GTATAATGAG
            1310       1320       1330       1340       1350:
   TCACTTCTTT GTTGTAAACT CTCCTTAGAA TTTCTTGGGA GAGGAACTGA
```

```
        1360       1370       1380       1390       1400
ACAGAACATT GATTTCCTAT GTGAGAGAAT TCTTAGAATT TAAATAAACC
        1410       1420       1430       1440       1450
TGTTGGTTAA ACTGAAACCA CAAAATTAGC ATTTTACTAA TCAGTAGGTT
        1460       1470       1480       1490       1500
TAAATAGCTT GGAAGCAAAA GTCTGCCATC ACCTTGATCA TCAACCCAGC
        1510       1520       1530       1540       1550
TTGCTGCTTC TTCCCAGTCT TGGGTTCAAG GTATTATGTA TACATATAAC
        1560       1570       1580       1590       1600
AAAATTTCTA TGATTTTCCT CTGTCTCATC TTTCATTCTT CACTAATACG
        1610       1620       1630       1640       1650
CAGTTGTAAC TTTTCTATGT GATTGCAAGT ATTGGTACTT TCCTATGATA
        1660       1670       1680       1690       1700
TACTGTTAAA AATATATTTG CAAATGTTGA TACTATCTAT CTCAGAGCTA
        1710       1720       1730       1740       1750
TAGGTGAAAA ATTAAATACT TTTATAAAGA CCAAATTGAT CATTTTTAAA
        1760       1770       1780       1790       1800
CGAAATTCTT ATATACTGAA AATGTAGATA CATAACTTCA GTATAGATTT
        1810       1820       1830       1840       1850
ATGGTAAAAT AATTTGAATC ATTTTTGTCA AATTCTGTAA AAAGTTGTCA
        1860       1870       1880       1890       1900
TACAGAATAA TTTATAATAT TTTTGTTTTC ATAGAAATAA CATTTCTGGT
        1910       1920       1930       1940       1950
AGAATATTTC AAGGCCATTT TTATTTTGTG TAATTAGGTT AATAAAATTA
        1960       1970       1980       1990       2000
ATTTTATAAA GGAAATGTCA ATGATAGACA ATTAGATATA AATGACTACT
        2010       2020       2030       2040       2050
TTTATAAAGA TGATTAAATT TGGATATTTG TAAGGATACA AATATATGAA
        2060       2070       2080       2090       2100
AACAGTAGAC TCATTTGGGG CCTATAAATA TGTCTTTTTT AACAAATGCA
        2110       2120       2130       2140       2150
GGTAGATTCT ACAGTTTGTA AACTGAAGCA GCCTATATAA AATAATCTGT
        2160       2170       2180       2190       2200
CATTAGTTTG CTGACTAAGG TATAAACCCT TCATGTATAA TCTAATTTTT
        2210       2220       2230       2240       2250
CTTATGTATC TGAAACGCAT TTTTCCAGCA CATATAATGT AGTATTTTTG
        2260       2270       2280       2290       2300
GGTCTTGCAA TTTAATGGAA CTCTAGGAGT CAAACGTGAT ATGTTTGACT
        2310       2320       2330       2340       2350
TATGATTCTG TTTAATCATC TTCATTCAGT CATGTCATGG ATATATCAAC
        2360       2370       2380       2390       2400
CCAGCAAAAT TAAGTAATAG CTAGATCCTT TTAAAAATTT AATGAAGGTT
        2410       2420       2430       2440       2450
AATAGTTTCT ACATAATGCA CAATGTTTTT CATGAAGACT CTGAAAGAGC
        2460       2470       2480       2490       2500
AGGCTAAAGG ATAAAGACAT TTTAAAAAAT TACAGATATT AAATGTAATT
        2510       2520       2530       2540       2550
TACCAGTGGG TTTTAGTTTA TCAATTTTAA CAAATCCAAT GATCTAAGAG
        2560       2570       2580       2590       2600
GAAATTTCTT TTTAATTTTT TTTGTAGTAT TTTAAAATTT GTAATATTTA
        2610       2620       2630       2640       2650
AATATTGATG CTTCTCTATT CCTCTGACAA AACCCTACTA TTACTTTCAG
        2660       2670       2680       2690       2700
GATCAAATGC TTTACTTTAA AGTGTAGTAA GATATTGGTA TTTCTTATCT
```

```
        2710        2720        2730        2740        2750
   TATATAAGCA  CTAAGCAAAA  TAATTTGAAT  GGTAAATATT  TATATTGAAG
        2760        2770        2780        2790        2800
   AGCAAAATTA  AAAACTAAAT  GAATAAAAAT  ATTACTTTCA  AGTGCAACAA
        2810        2820        2830        2840        2850
   CTTTTATCAT  AATATACTCC  TTTGTTGGAA  AAATTAAGAA  TTTTTTTTTC
        2860        2870        2880        2890        2900
   ATGAATCAAA  TTTTATTATA  AGACCTAACT  ATTTTATTTT  CTTACATAGA
        2910        2920        2930        2940        2950
   TCTTGACAAC  CATGAAACTT  CTCATCCTTA  CCTGTCTTGT  GGCTGTTGCT
        2960        2970        2980        2990        3000
   CTTGCCAGGC  CTGTGAGTAC  AGTAGAGAAT  TTAGAAGATT  CTAGATTCTT
        3010        3020        3030        3040        3050
   GTTTAAAGTC  ATCTCAAATG  CAATTTGATG  CAAGTCTCAT  CAAGTGCAAG
        3060        3070        3080        3090        3100
   ATATTGTAGT  CATAAAGAAT  TTGATGGTCT  CTAATTAGCT  ATTAAAGTGT
        3110        3120        3130        3140        3150
   TGATATTAAA  GCTATGGTAA  TCCTTCACAT  TTTGATCATT  ATTATTTAGT
        3160        3170        3180        3190        3200
   TTATTCAAAG  ACTTAACTCT  AAATAAAATT  TCTAACTTGA  AATATAAACA
        3210        3220        3230        3240        3250
   CCTCACAATT  AAAAATTTAA  AAAAAAAAGA  AATAAGGTAA  TTAACAATAC
        3260        3270        3280        3290        3300
   AATAAAGAGC  ATCAAAGAAG  GTAACTAGTC  TCTTTGCCTG  GGTCCATTAT
        3310        3320        3330        3340        3350
   AGGCTTAACA  TATTTGTAAA  CATATATATA  TCCAATATTG  TATTACCAAA
        3360        3370        3380        3390        3400
   TATACTGCTG  CTGCTGCTAA  GTCACTTCAG  TCGTGTCTGA  CTCTGTGCGA
        3410        3420        3430        3440        3450
   CACCATTGAT  GGCAGCCCAC  ACAGCTCTGC  CATCCCTGGG  ATTCTCCAGG
        3460        3470        3480        3490        3500
   CAAGAACACT  GGAGTGGGTT  GCCATTTCCT  TCTCCAATGC  ATGAAAGTGA
        3510        3520        3530        3540        3550
   AAAGTGAAAG  TGAAGTCTCT  CAGTCGTGTC  CGACTCCTAG  CAACCCCATG
        3560        3570        3580        3590        3600
   GACTGCCAGG  CTCCTCCATC  CATGGGATTT  TCCAGGCAAG  AGTACTGGAG
        3610        3620        3630        3640        3650
   TGGGTTGCCA  TTGCCTTCTC  CAACCAAATA  TACTATTAAA  CCCTATATTC
        3660        3670        3680        3690        3700
   CAGTGTATCC  ACTTTTGGAA  CCTAAATCAA  ACCCTCATTT  GAGATGGCTC
        3710        3720        3730        3740        3750
   ATGCCAACCA  ATATTTCCCA  AGGTACAGAA  AGTTGGGCTC  ATTCAGCTGA
        3760        3770        3780        3790        3800
   TTCAAAGATC  TAATAATTTG  GTCCTTGTAG  AAAGAAAAAC  TAGATAATGT
        3810        3820        3830        3840        3850
   AAAGTAACTT  AAGTTTCTTC  TCAAAAAACA  AATTCAGTTA  TTAATGTGAA
        3860        3870        3880        3890        3900
   ACAAAAAGTT  ATTCGTTCCT  TTGTGACCTC  TGCTGCTAAG  TCGCTTCAGT
        3910        3920        3930        3940        3950
   CGTGTCCGAC  TCTGTGCGAC  CCCATAGACA  GCAGCCCACA  AGGCTCCCCC
        3960        3970        3980        3990        4000
   ATCCCTGGGA  TTCTCCAAGC  AAGAACACTG  GAGTGGGTTG  CCATTTCCTT
```

19

```
        4010        4020        4030        4040        4050
CTCCAATGCA TGAAAGTGAG AAGGGAAAGT GAAGTCGCTC AGTCATGTCT
        4060        4070        4080        4090        4100
GACTCTTAGC GACCTCATGG ACTGCAGCCC ACCAGGCTCC TCTGTCCATG
        4110        4120        4130        4140        4150
GGATTTTCCA GGCAAGAGTA CTGGAGTGGG GTGCCATTGC CTTCTCCGTT
        4160        4170        4180        4190        4200
GGTGACCTCT AGATAATGAT AAATAAATAA ATAGGAATCA ACTGACAAGA
        4210        4220        4230        4240        4250
AAGTGATTCA AATAAGATAA TAGTTTTGGA TATTTGGACA CTCAAACTAT
        4260        4270        4280        4290        4300
CAAATATAGA TGAAAAGTT TCTGAAATGC TGAGATATTC TATTGTTAAA
        4310        4320        4330        4340        4350
CTCTTATTTT CTAAATTGTA AATAATGATT GAAGGATCAC TAATAATCCA
        4360        4370        4380        4390        4400
GCTTCTTAAC CAATAGAGTT CTGTCTGTGC TAAACCCTAA GCATCAAAAG
        4410        4420        4430
ATGGAAATAT CTGACAGTAA GTTACAAAAA AAGGATCC 3'
```

Anhang 2a

```
             10         20         30         40         50
5' TGCAGATCTT GACAACCATG AAACTTCTCA TCCTTACCTG TCTTGTGGCT
             60         70         80         90        100
   GTTGCTCTTG CCACTGAGAT CACCAGAATC CCTCTGTACA AAGGCAAGTC
            110        120        130        140        150
   TCTGAGGAAG GCGCTGAAGG AGCATGGGCT TCTGGAGGAC TTCCTGCAAA
            160        170        180        190        200
   AACAGCAGTA TGGCATCAGC AGCAAGTACT CCGGCTTCGG GGAGGTGGCC
            210        220        230        240        250
   AGCGTGCCCC TGACCAACTA CCTGGATAGT CAGTACTTTG GGAAAATCTA
            260        270        280        290        300
   CCTCGGGACC CCGCCCCAGG AGTTCACCGT GCTGTTTGAC ACTGGCTCCT
            310        320        330        340        350
   CTGACTTCTG GGTACCCTCT ATCTACTGCA AGAGCAATGC CTGCAAAAAC
            360        370        380        390        400
   CACCAGCGCT TCGACCCGAG AAAGTCGTCC ACCTTCCAGA ACCTGGGCAA
            410        420        430        440        450
   GCCCCTGTCT ATCCACTACG GGACAGGCAG CATGCAGGGC ATCCTGGGCT
            460        470        480        490        500
   ATGACACCGT CACTGTCTCC AACATTGTGG ACATCCAGCA GACAGTAGGC
            510        520        530        540        550
   CTGAGCACCC AGGAGCCCGG GGACGTCTTC ACCTATGCCG AATTTGACGG
            560        570        580        590        600
   AATCCTGGGG ATGGCCTACC CCTCGCTCGC CTCAGAGTAC TCGATACCCG
            610        620        630        640        650
   TGTTTGACAA CATGATGAAC AGGCACCTGG TGGCCCAAGA CCTGTTCTCG
            660        670        680        690        700
   GTTTACATGG ACAGGAATGG CCAGGAGAGC ATGCTCACGC TGGGGGCCAT
            710        720        730        740        750
   CGACCCGTCC TACTACACAG GGTCCCTGCA CTGGGTGCCC GTGACAGTGC
            760        770        780        790        800
   AGCAGTACTG GCAGTTCACT GTGGACAGTG TCACCATCAG CGGTGTGGTT
            810        820        830        840        850
   GTGGCCTGTG AGGGTGGCTG TCAGGCCATC CTGGACACGG GCACCTCCAA
            860        870        880        890        900
   GCTGGTCGGG CCCAGCAGCG ATATCCTCAA CATCCAGCAG GCCATTGGAG
            910        920        930        940        950
   CCACACAGAA CCAGTACGGT GAGTTTGACA TCGACTGCGA CAACCTGAGC
            960        970        980        990       1000
   TACATGCCCA CTGTGGTCTT TGAGATCAAT GGCAAAATGT ACCCACTGAC
           1010       1020       1030       1040       1050
   CCCCTCCGCC TATACCAGCC AGGACCAGGG CTTCTGTACC AGTGGCTTCC
           1060       1070       1080       1090       1100
   AGAGTGAAAA TCATTCCCAG AAATGGATAC TGGGGGATGT TTTCATCCGA
           1110       1120       1130       1140       1150
   GAGTATTACA GCGTCTTTGA CAGGGCCAAC AACCTCGTGG GGCTGGCCAA
           1160       1170       1180       1190       1200
   AGCCATCTGA GGAGTCAAGT GAATTCGCGG CCGCGTCGAC AAGCTTGAGA
           1210
   GCTCGGTACC 3'
```

21

Anhang 2b

```
              10         20         30         40         50
      5' GGTACCGAGC TCTCAAGCTT GTCGACGCGG CCGCGAATTC ACTTGACTCC
              60         70         80         90        100
         TCAGATGGCT TTGGCCAGCC CCACGAGGTT GTTGGCCCTG TCAAAGACGC
             110        120        130        140        150
         TGTAATACTC TCGGATGAAA ACATCCCCCA GTATCCATTT CTGGGAATGA
             160        170        180        190        200
         TTTTCACTCT GGAAGCCACT GGTACAGAAG CCCTGGTCCT GGCTGGTATA
             210        220        230        240        250
         GGCGGAGGGG GTCAGTGGGT ACATTTTGCC ATTGATCTCA AAGACCACAG
             260        270        280        290        300
         TGGGCATGTA GCTCAGGTTG TCGCAGTCGA TGTCAAACTC ACCGTACTGG
             310        320        330        340        350
         TTCTGTGTGG CTCCAATGGC CTGCTGGATG TTGAGGATAT CGCTGCTGGG
             360        370        380        390        400
         CCCGACCAGC TTGGAGGTGC CCGTGTCCAG GATGGCCTGA CAGCCACCCT
             410        420        430        440        450
         CACAGGCCAC AACCACACCG CTGATGGTGA CACTGTCCAC AGTGAACTGC
             460        470        480        490        500
         CAGTACTGCT GCACTGTCAC GGGCACCCAG TGCAGGGACC CTGTGTAGTA
             510        520        530        540        550
         GGACGGGTCG ATGGCCCCCA GCGTGAGCAT GCTCTCCTGG CCATTCCTGT
             560        570        580        590        600
         CCATGTAAAC CGAGAACAGG TCTTGGGCCA CCAGGTGCCT GTTCATCATG
             610        620        630        640        650
         TTGTCAAACA CGGGTATCGA GTACTCTGAG GCGAGCGAGG GGTAGGCCAT
             660        670        680        690        700
         CCCCAGGATT CCGTCAAATT CGGCATAGGT GAAGACGTCC CCGGGCTCCT
             710        720        730        740        750
         GGGTGCTCAG GCCTACTGTC TGCTGGATGT CCACAATGTT GGAGACAGTG
             760        770        780        790        800
         ACGGTGTCAT AGCCCAGGAT GCCCTGCATG CTGCCTGTCC CGTAGTGGAT
             810        820        830        840        850
         AGACAGGGGC TTGCCCAGGT TCTGGAAGGT GGACGACTTT CTCGGGTCGA
             860        870        880        890        900
         AGCGCTGGTG GTTTTTGCAG GCATTGCTCT TGCAGTAGAT AGAGGGTACC
             910        920        930        940        950
         CAGAAGTCAG AGGAGCCAGT GTCAAACAGC ACGGTGAACT CCTGGGGCGG
             960        970        980        990       1000
         GGTCCCGAGG TAGATTTTCC CAAAGTACTG ACTATCCAGG TAGTTGGTCA
            1010       1020       1030       1040       1050
         GGGGCACGCT GGCCACCTCC CCGAAGCCGG AGTACTTGCT GCTGATGCCA
            1060       1070       1080       1090       1100
         TACTGCTGTT TTTGCAGGAA GTCCTCCAGA AGCCCATGCT CCTTCAGCGC
            1110       1120       1130       1140       1150
         CTTCCTCAGA GACTTGCCTT TGTACAGAGG GATTCTGGTG ATCTCAGTGG
            1160       1170       1180       1190       1200
         CAAGAGCAAC AGCCACAAGA CAGGTAAGGA TGAGAAGTTT CATGGTTGTC
            1210
         AAGATCTGCA 3'
```

## Patentansprüche

1. Rekombinantes DNA-Konstrukt zur Expression von Proteinen in der Milchdrüse transgener Säugetiere und nachfolgender Sekretion in die Milch, **dadurch gekennzeichnet,** daß es die folgenden DNA-Bereiche in der angegebenen Reihenfolge und in funktioneller Verknüpfung zueinander enthält:
   (1) eine Transkriptions-Kontrollregion aus einem oder mehreren spezifisch in der Milchdrüse aktivierten Genen,

(2) ein erster DNA-Sequenzbereich, der nicht translatiert wird,

(3) eine DNA-Sequenz, die für ein Signalpeptid kodiert, das eine Sekretion in die Milch erlaubt,

(4) eine DNA-Sequenz, welche die genetische Information für ein heterologes Peptid oder ein Protein enthält und

(5) eine DNA-Sequenz, die eine Polyadenylierungsstelle und ein Transkriptions-Terminationssignal enthält.

2. DNA-Konstrukt nach Anspruch 1, **dadurch gekennzeichnet,** daß die Transkriptions-Kontrollregion aus einem oder mehreren Genen der Caseinfamilie stammt.

3. DNA-Konstrukt nach Anspruch 2, **dadurch gekennzeichnet,** daß die Transkriptions-Kontrollregion aus dem $\alpha$-$S_1$-Caseingen stammt.

4. DNA-Konstrukt nach einem der Ansprüche 1 bis 3, **dadurch gekennzeichnet,** daß der erste nicht-translatierte Bereich das Exon I aus einem Gen der Caseingenfamilie, vorzugsweise dem $\alpha$-$S_1$-Caseingen enthält.

5. DNA-Konstrukt nach einem der Ansprüche 1 bis 4, **dadurch gekennzeichnet,** daß der erste nicht-translatierte Bereich das Intron aus einem Gen der Caseingenfamilie, vorzugsweise dem $\alpha$-$S_1$-Caseingen enthält.

6. DNA-Konstrukt nach einem der Ansprüche 1 bis 5, **dadurch gekennzeichnet,** daß die für ein Signalpeptid kodierende DNA-Sequenz die Signalsequenz des zu exprimierenden Proteins oder die Signalsequenz eines in die Milch sekretierten Proteins ist.

7. DNA-Konstrukt nach Anspruch 6, **dadurch gekennzeichnet,** daß die Signalsequenz aus dem $\alpha$-$S_1$-Caseingen stammt.

8. DNA-Konstrukt nach einem der Ansprüche 1 bis 7, **dadurch gekennzeichnet,** daß es eine DNA-Sequenz (4) enthält, die für ein Peptid oder ein Protein in aktiver Form ohne Fusionsanteil kodiert.

9. DNA-Konstrukt nach einem der Ansprüche 1 bis 7, **dadurch gekennzeichnet,** daß es eine DNA-Sequenz (4) enthält, die für ein Peptid oder ein Protein in einer inaktiven Vorläuferform ohne Fusionsanteil kodiert.

10. DNA-Konstrukt nach Anspruch 8 oder 9, **dadurch gekennzeichnet,** daß es eine DNA-Sequenz (4) enthält, die für das Prorennin aus dem Rind oder den menschlichen IGF-I kodiert.

11. DNA-Konstrukt nach einem der Ansprüche 1 bis 10, **dadurch gekennzeichnet,** daß es zwischen den DNA-Sequenzen (4) und (5) einen zweiten nicht-translatierten Sequenzbereich enthält.

12. DNA-Konstrukt nach Anspruch 11, **dadurch gekennzeichnet,** daß der zweite nicht-translatierte DNA-Sequenzbereich den Exon/Intron-Übergang zwischen dem vorletzten Exon und dem letzten Intron einem Gen der Caseingenfamilie, vorzugsweise des $\alpha$-$S_1$-Caseingens enthält.

13. DNA-Konstrukt nach einem der Ansprüche 1 bis 12, **dadurch gekennzeichnet,** daß es an einem 3'-Ende noch zusätzliche nichttranskribierte und nicht-translatierte DNA-Sequenzen enthält.

14. DNA-Konstrukt nach einem der Ansprüche 1 bis 13, **dadurch gekennzeichnet,** daß es an seinem 5'- und 3'-Ende jeweils eine Restriktionsschnittstelle eines oder mehrerer Enzyme enthält, die innerhalb des DNA-Konstruktes keine Schnittstelle besitzen.

15. Rekombinanter Vektor, **dadurch gekennzeichnet,** daß er ein DNA-Konstrukt nach einem der Ansprüche 1 bis 14 enthält.

16. Verfahren zur Gewinnung von Proteinen aus der Milch transgener Tiere, **dadurch gekennzeichnet,** daß man eine rekombinante DNA nach einem der Ansprüche 1 bis 14 oder einem rekombinanten Vektor nach Anspruch 15 in die Keimbahn eines Tieres einbringt und das Protein auf übliche Weise aus der Milch des Tieres oder seiner weiblichen Nachkommen isoliert.

17. Verfahren nach Anspruch 16, **dadurch gekennzeichnet,** daß man eine rekombinante DNA nach einem der Ansprüche 1 bis 14 durch Mikroinjektion in die Keimbahn eines Tieres einbringt.

18. Verfahren nach Anspruch 16 oder 17, **dadurch gekennzeichnet,** daß man ein Tier verwendet, in dessen Milch kein endogenes, dem zu exprimierenden Protein homologes Protein vorliegt.

19. Verfahren nach Anspruch 16 oder 17, **dadurch gekennzeichnet,** daß man ein Tier verwendet, in dessen Milch ein endogenes, dem zu exprimierenden Protein vollständig homologes Protein vorliegt.

20. Verfahren nach Anspruch 16 zur Gewinnung eines aus zwei oder mehreren verschiedenen Untereinheiten zusammengesetzten Proteinkomplexess, **dadurch gekennzeichnet,** daß man transgene Tiere verwendet, die zwei oder mehrere verschiedene DNA-Konstrukte nach einem der Ansprüche 1 bis 13 in ihrem Genom integriert besitzen.

21. Verfahren zur Herstellung transgener Tiere, die zwei oder mehrere verschiedene Fremdgene in ihrem Genom integriert besitzen, **dadurch gekennzeichnet,** daß man
(1) zwei transgene Tiere der gleichen Art, die aber jeweils ein oder mehrere verschiedene Fremdgene in ihrem Genom integriert besitzen, auf übliche Weise herstellt, diese Tiere oder deren Nachkommen miteinander kreuzt und aus den Nachkommen diejenigen transgenen Tiere auswählt, welche gleichzeitig für die verschiedenen Fremdgene beider Elterntiere positiv sind, oder
(2) ein transgenes Tier auf übliche Weise herstellt, wobei man ein Fremdgen in die Keimbahn eines Tieres einbringt, das bereits eines oder mehrere andere Fremdgene in seinem Genom integriert besitzt.

22. Verfahren nach Anspruch 21, **dadurch gekennzeichnet,** daß man zum Einbringen eines Fremdgens ein DNA-Konstrukt nach einem der Ansprüche 1 bis 14 oder einen Vektor nach Anspruch 15 verwendet.

23. Milch eines transgenen Tieres, das ein oder mehrere rekombinante DNA-Konstrukte nach einem der Ansprüche 1 bis 14 in seinem Genom integriert enthält.

24. Rekombinantes DNA-Konstrukt, **dadurch gekennzeichnet,** daß es die DNA-Bereiche (1), (2), (3) und (5) nach einem der Ansprüche 1 bis 7 und anstelle des DNA-Bereichs (4), welcher die genetische Information für ein heterologes Peptid oder ein Protein enthält, eine Klonierungsstelle enthält.

25. DNA-Konstrukt nach Anspruch 24, **dadurch gekennzeichnet,** daß es einen oder mehrere weitere DNA-Bereiche nach einem der Ansprüche 11 bis 14 enthält.

26. DNA-Konstrukt nach Anspruch 24 oder 25, **dadurch gekennzeichnet,** daß die Klonierungsstelle die Schnittstellen für mehrere Restriktionsenzyme umfaßt.

24

**Fig. 1**

λ319

λ84

λ122

p26

p19

p84

p86

10kb

B = BamHI
E = EcoRI
H = HindIII
S = SalI
X = XbaI

*Fig. 2*

B = BamHI

E = EcoRI

H = HindIII

S = SalI

EP 0 451 823 A2

_Fig. 3.1_

*Fig. 3.2*

*Fig. 3.3*

*Fig. 3. 4*

Fig. 3.5

*Fig. 3. 6*

*Fig. 3.7*

*Fig. 3.8*

*Fig. 3.9*

| IF1 | IF3 | IF5 |
| + | + | + |
| IF2 | IF4 | IF6 |

Hybridi-sierung    Hybridi-sierung    Hybridi-sierung

| IF1 | + | IF3 | + | IF5 |
| IF2 | | IF4 | | IF6 |

Ligation

| IF1 | IF3 | IF5 |
| IF2 | IF4 | IF6 |

*Fig. 3.10 a*

EP 0 451 823 A2

```
                                                _____ Signalpeptid _____
IF1  │GAT CTT GAC AAC CAT GAA ACT TCT CAT CCT TAC CTG TCT TGT  IF1
IF2    │AA CTG TTG GTA CTT TGA AGA GTA GGA ATG GAC AGA ACA  IF2

     _____ IGF-I _____
IF1  GGC TGT TGC TCT TGC CGG CCC CGA GAC CCT GTG CGG GGC CGA  IF1
IF2  CCG ACA ACG AGA ACG GCC GGG GCT CTG GGA CAC GCC CCG GCT  IF2


IF1  GCT GGT GGA CGC│CCT GCA GTT CGT GTG CGG CGA CCG CGG CTT  IF3
IF2  CGA CCA CCT GCG GGA CGT CAA GCA│CAC GCC GCT GGC GCC GAA  IF4


IF3  CTA CTT CAA CAA GCC CAC CGG CTA CGG CTC CAG CTC CCG CCG  IF3
IF4  GAT GAA GTT GTT CGG GTG GCC GAT GCC GAG GTC GAG GGC GGC  IF4


IF3  GGC CCC CCA GAC CGG CAT CGT GGA CGA│GTG CTG CTT CCG GAG  IF5
IF4  CCG GGG GGT CTG GCC GTA GCA CCT GCT CAC GAC GAA GGC│CTC  IF6


IF5  CTG CGA CCT GCG CCG GCT GGA GAT GTA CTG CGC CCC CCT GAA  IF5
IF6  GAC GCT GGA CGC GGC CGA CCT CTA CAT GAC GCG GGG GGA CTT  IF6

     _____ IGF-I _____              _____ 3'-Caseinbereich _____
IF5  GCC CGC CAA GTC CGC CTG AGG AGT CAA GTG AAT TCC GCG GGA  IF5
IF6  CGG GCG GTT CAG GCG GAC TCC TCA GTT CAC TTA AGG CGC CCT  IF6


IF5  │TCC GTC GAC│                                              IF5
IF6  │AGG CAG CTG TTA A│                                         IF6
```

**Fig. 3.10b**

Fig. 3.11

Fig. 3.12

## Fig. 3.13

Fig. 3.14